# EUROPEAN PATENT APPLICATION

(11) **EP 4 442 256 A1**
(43) Date of publication of application: **09.10.2024**
(21) Application number: 23166579.5
(22) Date of filing: 04.04.2023
(51) Int. Cl.: A61K 9/51, C12N 15/11, A61K 39/00

(54) **LIPID NANOPARTICLE COMPOSITIONS**

(71) Applicant: Københavns Universitet, 2200 København N (DK); Statens Serum Institut, 2300 Copenhagen S (DK)
(72) Inventor: FOGED, Camilla, 2200 København N (DK); LOKRAS, Abhijeet Girish, 2200 København N (DK); THAKUR, Aneesh, 2200 København N (DK); FRANZYK, Henrik, 2200 København N (DK); CHRISTENSEN, Dennis Engelmann, 2300 København S (DK)
(74) Representative: Plougmann Vingtoft a/s

(57) **Abstract**

The present invention relates to the field of lipid nanoparticles (LNPs). In particular, the present invention relates to an LNP composition comprising a cationic or cationically ionisable lipid or lipid-like material, a helper lipid, a lipopolymer, and a monomycoloyl glycerol (MMG) analogue. The LNP composition is particularly useful as a vaccine composition.

## Description

### Technical field of the invention

The present invention relates to the field of lipid nanoparticles (LNPs). In particular, the present invention relates to an LNP composition comprising a cationic or cationically ionisable lipid or lipid-like material, a helper lipid, a lipopolymer, and a monomycoloyl glycerol (MMG) analogue. The LNP composition is particularly useful as a vaccine composition.

### Background of the invention

RNA therapeutics, including mRNA vaccines, are susceptible to nuclease degradation and cannot permeate the cell membrane due to their large size and negative charge and thus require a delivery system. A promising delivery system in this regard is lipid nanoparticles (LNPs). Profylactic vaccines based on mRNA-loaded LNPs (mRNA-LNPs) have proven highly effective against infectious disease, as demonstrated by the mRNA-LNP vaccines developed during the worldwide coronavirus disease 2019 (COVID-19) pandemic. Despite the success of the COVID-19 mRNA vaccines, first-generation mRNA vaccines display several weaknesses. For example, the LNP design is based on the LNP technology initially developed for systemic liver targeting of short interfering RNA (siRNA) used in the drug Onpattro^{®}, which was approved in 2018 for the treatment of polyneuropathies induced by hereditary transthyretin amyloidosis. Hence, there is a need to: (i) engineer RNA delivery systems that more precisely target the immune system, and (ii) optimise delivery systems specifically as antigen-encoding RNA carriers and adjuvants.

WO 2021/148511 A1 relates to the field of LNPs; more specifically comprising an ionisable lipid, a phospholipid, a sterol, a PEG lipid, and one or more nucleic acids. Said prior art document discloses the use of the LNPs for immunogenic delivery of nucleic acid molecules, specifically mRNA; thereby making them highly suitable for use in vaccines, such as for the treatment of cancer or infectious diseases. Although the LNPs of WO 2021/148511 A1 are developed for mRNA delivery, nothing in their design specifically targets the immune system such as by the incorporation of a ligand for a pattern-recognition receptor (PRR), e.g., a pathogen-associated molecular pattern (PAMP). Hence, an improved LNP composition would be advantageous, and in particular an LNP composition with an improved immunogenicity would be advantageous.

### Summary of the invention

Thus, an object of the present invention relates to a lipid nanoparticle (LNP) composition with an improved immunogenicity. In particular, it is an object of the present invention to provide an LNP composition that solves the above-mentioned problems of the prior art, is colloidally stable, and displays efficient intracellular delivery of nucleic acids.

Thus, one aspect of the invention relates to a lipid nanoparticle (LNP) composition comprising a cationic or cationically ionisable lipid or lipid-like material, a helper lipid, a lipopolymer, and a monomycoloyl glycerol (MMG) analogue,
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3",3‴-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-sn-glycero-3-phosphocholine (DOPC), 1,2-dioleoyl-sn-glycero-3-phospho-(1'-rac-glycerol) (DOPG), 1,2-dipalmitoyl-sn-glycero-3-phosphocholine (DPPC), 1,2-distearoyl-*sn-*glycero-3-phosphocholine (DSPC), 1,2-dipalmitoyl-sn-glycero-3-phosphoglycerol (DPPG), 1,2-dimyristoyl-sn-glycero-3-phosphocholine (DMPC), 1,2-diacyl-3-*O*-β-D-galactosyl-sn-glycerol (MGDG), 1-stearoyl-2-oleoyl-sn-glycero-3-phosphocholine (SOPC), 1,2-diacyl-3-O-(α-D-galactosyl1-6)-α-D-galactosyl-sn-glycerol (DGDG), and sulfoquinovosyldiacylglycerol (SQDG), or any mixture thereof, and
wherein the lipopolymer is a polyethylene glycol (PEG)- or polysarcosine-lipid conjugate or a PEG- or polysarcosine-lipid like conjugate, or any mixture thereof.

Another aspect relates to a vaccine composition comprising the lipid nanoparticle (LNP) composition according to the present invention and at least one nucleic acid encoding an antigen. Yet another aspect relates to said vaccine composition for use in the prevention and/or treatment of an infectious disease.

An aspect relates to a process for obtaining the lipid nanoparticle (LNP) composition according to the invention, said process comprising the steps of:
a) Providing a cationic or cationically ionisable lipid or lipid-like material, a helper lipid, a lipopolymer, a monomycoloyl glycerol (MMG) analogue, and at least one nucleic acid;
b) Dissolving the cationic or cationically ionisable lipid or lipid-like material, the helper lipid, the lipopolymer, and the MMG analogue of step a) in an organic solvent comprising ethanol, preferably absolute ethanol with a purity close to 100%, thereby providing an organic phase;
c) Diluting the at least one nucleic acid of step a) in an aqueous solvent comprising a buffer with a pH within the range of 3 to 7.8, thereby providing an aqueous phase;
d) Mixing the organic phase of step b) with the aqueous phase of step c) to obtain lipid nanoparticles (LNPs) by nanoprecipitation;
e) Performing filtration, preferably tangential flow filtration or dialysis, of the LNPs of step d) to obtain an LNP composition;

wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3",3‴-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-sn-glycero-3-phosphocholine (DOPC), 1,2-dioleoyl-sn-glycero-3-phospho-(1'-rac-glycerol) (DOPG), 1,2-dipalmitoyl-sn-glycero-3-phosphocholine (DPPC), 1,2-distearoyl-*sn-*glycero-3-phosphocholine (DSPC), 1,2-dipalmitoyl-sn-glycero-3-phosphoglycerol (DPPG), 1,2-dimyristoyl-sn-glycero-3-phosphocholine (DMPC), 1,2-diacyl-3-*O*-β-D-galactosyl-sn-glycerol (MGDG), 1-stearoyl-2-oleoyl-sn-glycero-3-phosphocholine (SOPC), 1,2-diacyl-3-*O*-(α-D-galactosyl1-6)-α-D-galactosyl-*sn-*glycerol (DGDG), and sulfoquinovosyldiacylglycerol (SQDG), or any mixture thereof,
wherein the lipopolymer is a polyethylene glycol (PEG)- or polysarcosine-lipid conjugate or a PEG- or polysarcosine-lipid like conjugate, or any mixture thereof, and
wherein the monomycoloyl glycerol (MMG) analogue is selected from the group consisting of MMG-1, MMG-2, MMG-3, MMG-4, MMG-5, MMG-6, and MMG-7, or any mixture thereof.

Another aspect relates to a lipid nanoparticle (LNP) composition obtained using the process of the present invention.

### Brief description of the figures

**Figure 1A** shows the *FLuc* mRNA entrapment (%) of different LNP formulations, wherein C12-200, MMG-1, DOPE, cholesterol, and DMPE-PEG₂₀₀₀ were either dissolved in an organic phase consisting of absolute ethanol *(grey bars),* or dissolved in an organic phase consisting of 90% ethanol with citrate buffer (10 mM, pH 3) *(black bars).*
**Figure 1B** shows cryoTEM images of *FLuc* mRNA-loaded C12-200 LNPs.
**Figure 1C** shows cryoTEM images of *FLuc* mRNA-loaded C12-200 MMG 23 LNPs.
The black arrows indicate the edge surface C12-200 MMG 23.
**Figure 1D** shows cryoTEM images of *FLuc* mRNA-loaded C12-200 MMG 47 LNPs.
The black arrows indicate the faceted morphology of C12-200 MMG 47.
**Figure 2** shows representative whole-body images of female BALB/c mice injected subcutaneously at the base of the tail with *Fluc* mRNA-loaded C12-200 LNP (1-4) and C12-200 MMG 47 (5-8) formulations. Representative whole-body images were taken from the prone and supine position at 6 h after dosing. The scale represents radiance (ranging from 0.1 - 2.0 × 10⁸) at the site of injection and in the liver.
**Figure 3** shows the bioluminescence signals (total flux) (supine position) quantified at the site of injection (SOI) at various time points post LNP injection.
The dotted line represents the background, i.e., the total flux of mice treated with PBS and D-luciferin only. Data points represent mean values ± SD (n = 4-5).
**Figure 4** shows the bioluminescence signals (total flux) (supine position) quantified at the site of injection (SOI). The dotted line represents the background, i.e., the total flux of PBS and D-luciferin treated mice. Data points represent mean values ± SD (n = 4-5). ***p < 0.001, and ****p < 0.0001 via one-way ANOVA with Dunnett's multiple comparisons test.
**Figure 5** shows the percentages of cytokine-producing (IFN-γ, IL-2, IL-4, TNF-a and IL-17A) MHC-I Tet⁺CD8⁺CD44⁺ T cells after restimulating lymph node (LN) cells with OVA₂₅₇₋₂₆₄ peptide, in mice vaccinated with C12-200 LNPs or MMG-1-modified C12-200 LNPs (mean values ± SD, n = 6). One-way ANOVA with Tukey's Multiple Comparison test. *p* values; *p < 0.05, ***p* < 0.01, ***p < 0.001, and ****p < 0.0001.
**Figure 6** shows the percentages of cytokine-producing (IFN-γ, IL-2, IL-4, TNF-a and IL-17A) MHC-II Tet⁺CD4⁺CD44⁺ T cells after restimulating LN cells with OVA₃₂₃₋₃₃₉ peptide, in mice vaccinated with C12-200 LNPs or MMG-1-modified C12-200 LNPs (n = 6, mean ± SD). One-way ANOVA with Tukey's Multiple Comparison test. *p* values; *p < 0.05.
**Figure 7A** shows the numbers of OVA protein-specific T_{FH} cells in mice vaccinated with LNPs (n = 6, mean values ± SD). One-way ANOVA with Tukey's Multiple Comparison test.
**Figure 7B** shows the numbers of OVA₂₅₇₋₂₆₄ peptide-specific T_{FH} cells in mice vaccinated with LNPs (n = 6, mean values ± SD). One-way ANOVA with Tukey's Multiple Comparison test.
**Figure 7C** shows the numbers of OVA₃₂₃₋₃₃₉ peptide-specific T_{FH} cells in mice vaccinated with LNPs (n = 6, mean values ± SD). One-way ANOVA with Tukey's Multiple Comparison test.
**Figure 7D** shows the numbers of OVA protein-specific GC B cells in mice vaccinated with LNPs (n = 6, mean values ± SD). One-way ANOVA with Tukey's Multiple Comparison test. *p*-values; *p < 0.05.
**Figure 7E** shows the numbers of OVA₂₅₇₋₂₆₄ peptide-specific GC B cells in mice vaccinated with LNPs (n = 6, mean values ± SD). One-way ANOVA with Tukey's Multiple Comparison test.
**Figure 7F** shows the numbers of OVA₃₂₃₋₃₃₉ peptide-specific GC B cells in mice vaccinated with LNPs (n = 6, mean values ± SD). One-way ANOVA with Tukey's Multiple Comparison test.
**Figure 7G** shows mid-point titers (log EC₅₀ values) of OVA-specific IgG determined by endpoint dilution ELISA using sera of immunised mice collected 6 weeks after prime immunisation. Bars represent mean values ± SD, n = 6. One-way ANOVA with Tukey's Multiple Comparison test. *p*-values; *p < 0.05, ***p <* 0.01, ***p < 0.001, and ****p < 0.0001.
**Figure 7H** shows mid-point titers (log EC₅₀ values) of OVA-specific IgG1 determined by endpoint dilution ELISA using sera of immunised mice collected 6 weeks after prime immunisation. Bars represent mean values ± SD, n = 6. One-way ANOVA with Tukey's Multiple Comparison test. *p*-values; *p < 0.05, **p < 0.01, ***p < 0.001, and ****p < 0.0001.
Figure 71- shows mid-point titers (log EC₅₀ values) of OVA-specific IgG2c determined by endpoint dilution ELISA using sera of immunised mice collected 6 weeks after prime immunisation. Bars represent mean values ± SD, n = 6. One-way ANOVA with Tukey's Multiple Comparison test. *p*-values; *p < 0.05, **p < 0.01, ***p < 0.001, and ****p < 0.0001.

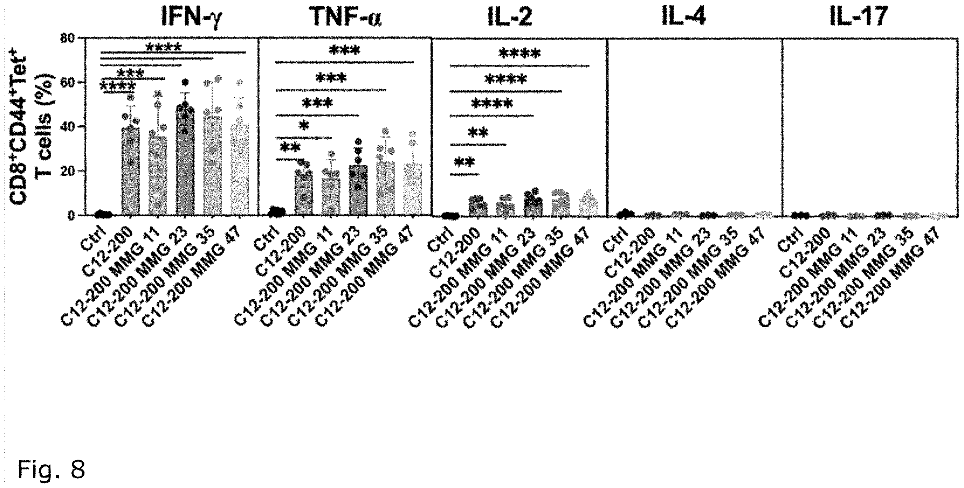

**Figure 8** shows the percentages of cytokine-producing (IFN-γ, IL-2, IL-4, TNF-a and IL-17A) OVA₂₅₇₋₂₆₄ peptide-specific MHC-I Tet⁺CD8⁺CD44⁺ T cells in mice vaccinated with LNPs (mean values ± SD, n = 6). One-way ANOVA with Tukey's Multiple Comparison test. *p*-values; *p < 0.05, **p < 0.01, ***p < 0.001, and ****p < 0.0001.

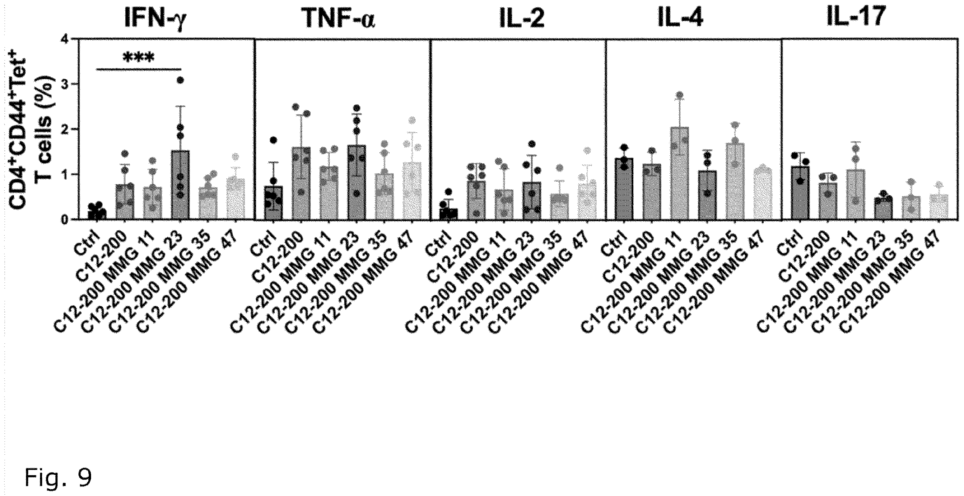

**Figure 9** shows the percentages of cytokine-producing (IFN-γ, IL-2, IL-4, TNF-a and IL-17A) OVA₃₂₃₋₃₃₉ peptide-specific MHC-II Tet⁺CD4⁺CD44⁺ T cells in the spleen of mice vaccinated with LNPs (mean values ± SD, n = 6). One-way ANOVA with Tukey's Multiple Comparison test. *p*-values; ***p < 0.001.

The present invention will now be described in more detail in the following.

### Detailed description of the invention

### Definitions

Prior to discussing the present invention in further details, the following terms and conventions will first be defined:

### Lipid nanoparticle (LNP)

In the present context, the term "lipid nanoparticle", abbreviated LNP, relate to a nanoparticle composed of lipids. A lipid nanoparticle is typically spherical with an average diameter between 10 and 1000 nanometers.

LNPs typically comprise four components: a cationic or cationically ionisable lipid or lipid-like material, a helper lipid, cholesterol, and a lipopolymer. The cationic or cationically ionisable lipid or lipid-like material plays a crucial role in protecting mRNA against nucleases and for its intracellular release. Cationically ionisable lipids are cationic at acidic pH, where they form electrostatic complexes with mRNA, but are neutral at physiological pH to minimise cellular toxicity, whereas cationic lipids or lipid-like material are cationically charged regardless of their surroundings. The structural helper lipid, e.g., the phospholipids distearoylphosphatidylcholine (DSPC) and dioleoylphosphoethanolamine (DOPE), supports bilayer stability during storage and circulation and improves mRNA encapsulation. Cholesterol plays several roles, including filling gaps in the particles, limiting LNP-protein interactions, maintains membrane integrity, and possibly promoting membrane fusion. The lipopolymer, which may be PEGylated lipids or polysarcosine-lipid conjugates, contributes to colloidal stabilisation of the LNPs by forming a hydrophilic steric barrier at the surface of LNPs, which prevents aggregation in formulation.

### Cationic or cationically ionisable lipid or lipid-like material

In the present context, the term "cationic or cationically ionisable lipid or lipid-like material" refer to lipids or lipid-like materials that are either permanently positively charged (cationic) or becomes positively charged whenever the pH changes (cationically ionisable). That is an "ionisable lipid or lipid-like material" is a lipid that is positively charged at acidic pH and neutral at physiological pH (~7.4). Ionisable lipids or lipid-like materials are positively charged at acidic pH, where they are used to condense and load RNAs into LNPs, but are neutral at physiological pH to minimise toxicity. They are protonated in the acidic endosome after cellular uptake, and interact with anionic endosomal phospholipids to form cone-shaped ion pairs that are not compatible with a lipid bilayer. These cationic-anionic lipid pairs drive the transition from a bilayer structure to an inverted hexagonal H_{II} phase, which is suggested to facilitate membrane fusion/disruption, endosomal escape and cargo release into the cytosol.

The terms "lipid" and "lipid-like material" are broadly defined herein as molecules, which comprise one or more hydrophobic moieties or groups and optionally also one or more hydrophilic moieties or groups. Molecules comprising hydrophobic moieties and hydrophilic moieties are also frequently denoted as amphiphiles. Lipids are usually poorly soluble in water. In an aqueous environment, the amphiphilic nature allows the molecules to self-assemble into organised structures and different phases. One of those phases consists of lipid bilayers, as they are present in vesicles, unilamellar/multilamellar liposomes, or membranes in an aqueous environment. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). The hydrophilic groups may comprise polar and/or charged groups and include carbohydrates, phosphate, carboxylic, sulfate, amino, sulfhydryl, nitro, hydroxyl, and other like groups.

### Lipid-like material or lipid-like conjugate

The term "lipid-like material", "lipid-like compound", "lipid-like conjugate", "lipid-like molecule" or "lipidoid" relates to substances that structurally and/or functionally relate to lipids but may not be considered as lipids in a strict sense. For example, the term includes compounds that are able to form amphiphilic layers as they are present in vesicles, unilamellar/multilamellar liposomes, or membranes in an aqueous environment and includes surfactants, or synthesised compounds with both hydrophilic and hydrophobic moieties. Generally speaking, the term refers to molecules, which comprise hydrophilic and hydrophobic moieties with different structural organisation, which may or may not be similar to that of lipids. As used herein, the term "lipid" is to be construed to cover both lipids and lipid-like materials unless otherwise indicated herein or clearly contradicted by context.

### Helper lipid

Helper lipids are a class of lipid molecules that increase particle stability and fluidity of LNPs. Several classes of molecules can be used as helper lipids such as phospholipids as exemplified by DSPC, DOPE, and sterols exemplified by cholesterol.

### Lipopolymer

In the present context, the term "lipopolymer" is any polymer covalently linked to a lipid (fatty acid or steroid) moiety such as PEGylated lipids or polysarcosine lipids.

### Monomycoloyl glycerol (MMG) and MMG analogue

The mycobacterial cell wall lipid monomycoloyl glycerol (MMG) or an analogue thereof is a glycerolipid.

Glycerolipids like MMG possess immunopotentiating properties and can enhance immune responses. MMG is too toxic for human use. Therefore, well-tolerated synthetic analogues of MMG have been developed.

The synthetic analogue, referred to as MMG-1, consists of a hydrophilic glycerol headgroup and a lipid acid, displaying two hydrophobic saturated C14/C15 alkyl tails, linked via an ester bond. Furthermore, an array of MMG analogues, differing in the alkyl chain lengths (MMG-2; C16/C17, MMG-3; C10/C11, and MMG-4; C6/C7), or with respect to the stereochemistry of the headgroup (MMG-5; 2S) and the lipid tail (MMG-6, MMG-7), has been designed.

MMG is preferably the synthetically manufactured glycerolipid, MMG-1. Monomycoloyl glycerol-1 (MMG-1) is a shorter synthetic analogue of the mycobacterial cell wall lipid, monomycoloyl glycerol (MMG). MMG-1 binds to the pattern-recognition receptor (PRR) C-type lectin-type receptor (CLR) called Mincle. Upon incorporation into liposomes, MMG-1 has been shown to display an immunomodulatory effect via activation of dendritic cells (DCs) resulting in increased secretion of the Th1 cytokine interferon gamma (IFN-γ) and the Th17 cytokine interleukin-17 (IL-17). The chemical structure of the preferred MMG analogue is 3-hydroxy-2-tetradecyl-octadecanoic acid-2,3-dihydroxypropyl ester, preferably the (2R)-2,3-dihydroxypropyl-3-hydroxy-2-tetradecyloctadecanoate diastereomer. Both MMG-6 and MMG-7 adopt an inverse hexagonal phase (Hu), which might enable higher endosomal escape and thereby a higher degree of nucleic acid cargo release than other MMG variants. Thus, both MMG-6 and MMG-7 are preferred analogues to use in the present invention.

### Cholesterol

Cholesterol is a sterol with the IUPAC name (3S,8S,9S,10R,13R,14S,17R)-10,13-dimethyl-17-[(2R)-6-methylheptan-2-yl]-2,3,4,7,8,9,11,12,14,15,16,17-dodecahydro-1*H*-cyclopenta[a]phenanthren-3-ol. Cholesterol is biosynthesised by all animal cells and constitutes an essential structural component of animal cell membranes by providing stability. In LNPs, cholesterol plays several roles including filling gaps in the particles, limiting LNP-protein interactions, maintains membrane integrity, and possibly promoting membrane fusion.

### Nucleic acid

In the present context, the term "nucleic acid" is a deoxyribonucleic acid (DNA) or preferably a ribonucleic acid (RNA), more preferably mRNA. Nucleic acids may also be referred to as a "nucleic acid cargo", since they do not form a part of the LNP carrier but instead are loaded into said LNP. Nucleic acids include, but are not limited to, genomic DNA, plasmid DNA, cDNA, mRNA, and recombinantly produced or chemically synthesised molecules. A nucleic acid according to the invention may be in the form of a molecule, which is single stranded or double stranded, and linear or closed covalently to form a circle. A nucleic acid can be employed for introduction into cells, i.e. transfection of cells, for example, in the form of RNA, which can be prepared enzymatically by *in vitro* transcription from a DNA template. The nucleic acid may be chemically modified by comprising one or more chemically modified nucleosides, such as pseudouridine, an N1-methyl pseudouridine, or a nucleoside with a 2'-O-methylation. Furthermore, the nucleic acid may also comprise phosphodiester bonds, phosphorothioate bonds, or a mixture thereof. The RNA can, moreover, be modified before application by stabilising sequences, capping, and/or polyadenylation.

### RNA molecule

In the context of the present invention, the term "RNA molecule" relates to a molecule, which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double-stranded RNA, single-stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesised nucleotides or deoxynucleotides.

### Organic phase

The term "organic phase" refers to an organic solvent, in which lipids, such as a cationic or cationically ionisable lipid or lipid-like material, a helper lipid, a lipopolymer, an MMG analogue and optionally cholesterol are dissolved. The organic solvent is ethanol, preferably absolute ethanol with a purity close to 100%.

### Aqueous phase

The term "aqueous phase" refers to an aqueous solvent in which at least one nucleic acid is dissolved. The aqueous solvent is a buffer, preferably with a pH of 3-7.8.

### Buffer

A buffer (more precisely, pH buffer or hydrogen ion buffer) is an acid or a base aqueous solution consisting of a mixture of a weak acid and its conjugate base, or vice versa. Its pH changes very little when a small amount of strong acid or base is added to it. Buffer solutions are used as a means of maintaining pH at a nearly constant value in a wide variety of chemical applications. In a preferred embodiment, the buffer is a citrate buffer. A citrate buffer is a buffered mixture of sodium citrate and citric acid. Citrate buffers can be used for RNA isolation, due to their ability to prevent base hydrolysis and magnesium ion-dependent RNA fragmentation by acting as chelators. In a preferred embodiment of the present invention, the pH of the citrate buffer is in the range of 3.0-4.0.

### Nanoprecipitation

Nanoprecipitation is the process, which is used to generate LNPs by mixing an organic phase comprising different lipids solubilised in an organic solvent with an aqueous phase comprising at least one nucleic acid solubilised in an aqueous phase. Nanoprecipitation can be performed by means of, but is not limited to, mixing the two phases using pipette mixing, a T-mixer, microfluidic mixing, or impingement jet mixers.

### Microfluidic mixing

Microfluidic mixing can be used to achieve a thorough and rapid mixing of multiple solvents in microscale devices. In the present invention, microfluidic mixing was used to generate LNPs by mixing an organic phase containing different lipids solubilised in an organic solvent with an aqueous phase containing at least one nucleic acid solubilised in an aqueous solvent. Said phases are injected separately into a microfluidic chip containing small channels with sizes of ten to hundreds of micrometers.

### Flow rate ratio

The term "flow rate ratio", abbreviated FRR, is in the present context used in regards to microfluidic mixing, wherein the flow rate ratio is the ratio between the flow rate of the aqueous phase and the flow rate of the organic phase. Hence, a flow rate ratio of 3:1 means that the aqueous phase flows through the microfluidic mixing chip three times faster than the organic phase.

### Total flow rate

In the present context, the term "total flow rate" is used in relation to microfluidic mixing, wherein said term denotes the collective flow rate of the aqueous phase and the organic phase in the microfluidic mixing chip.

### Antigen

In the present context, the term "antigen" refers to a molecule, such as an immunogenic peptide, that can induce an immune response. The immune response generated by the antigen may be B-cell driven (antibody-mediated immune response) and/or T-cell driven (cellular immune response).

### Administration

The term "administration" in the context of the present invention means administration in various modes either by systemic administration, such as intramuscular, subcutaneous, intradermal or intraperitoneal injection, or in delivery formulation or devices for e.g. topical-, intradermal-, intranasal-, sublingual-, oral- or pulmonary administration.

The LNP and/or vaccine composition according to the present invention is typically administered by parenteral administration, more typically by subcutaneous or intramuscular injection in the range of once per two weeks, to once or twice per month, to once or twice per year.

### A lipid nanoparticle composition

An object of the present invention relates to a lipid nanoparticle (LNP) composition with an improved immunogenicity, which is colloidally stable, and displays efficient intracellular delivery of nucleic acids. Thus, an aspect of the present invention relates to a lipid nanoparticle (LNP) composition comprising a cationic or cationically ionisable lipid or lipid-like material, a helper lipid, a lipopolymer, and a monomycoloyl glycerol (MMG) analogue,
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-sn-glycero-3-phosphocholine (DOPC), 1,2-dioleoyl-sn-glycero-3-phospho-(1'-rac-glycerol) (DOPG), 1,2-dipalmitoyl-*sn*-glycero-3-phosphocholine (DPPC), 1,2-distearoyl-*sn-*glycero-3-phosphocholine (DSPC), 1,2-dipalmitoyl-*sn*-glycero-3-phosphoglycerol (DPPG), 1,2-dimyristoyl-*sn*-glycero-3-phosphocholine (DMPC), 1,2-diacyl-3-*O*-β-D-galactosyl-*sn*-glycerol (MGDG), 1-stearoyl-2-oleoyl-sn-glycero-3-phosphocholine (SOPC), 1,2-diacyl-3-*O*-(α-D-galactosyl1-6)-α-D-galactosyl-sn-glycerol (DGDG), and sulfoquinovosyldiacylglycerol (SQDG), or any mixture thereof, and
wherein the lipopolymer is a polyethylene glycol (PEG)- or polysarcosine-lipid conjugate or a PEG- or polysarcosine-lipid like conjugate, or any mixture thereof.

An alternative aspect of the present invention relates to a lipid nanoparticle (LNP) composition consisting of a cationic or cationically ionisable lipid or lipid-like material, a helper lipid, a lipopolymer, and a monomycoloyl glycerol (MMG) analogue,
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-*sn*-glycero-3-phosphocholine (DOPC), 1,2-dioleoyl-*sn*-glycero-3-phospho-(1'-*rac*-glycerol) (DOPG), 1,2-dipalmitoyl-sn-glycero-3-phosphocholine (DPPC), 1,2-distearoyl-*sn-*glycero-3-phosphocholine (DSPC), 1,2-dipalmitoyl-sn-glycero-3-phosphoglycerol (DPPG), 1,2-dimyristoyl-sn-glycero-3-phosphocholine (DMPC), 1,2-diacyl-3-*O*-β-D-galactosyl-sn-glycerol (MGDG), 1-stearoyl-2-oleoyl-sn-glycero-3-phosphocholine (SOPC), 1,2-diacyl-3-*O*-(α-D-galactosyl1-6)-α-D-galactosyl-*sn-*glycerol (DGDG), and sulfoquinovosyldiacylglycerol (SQDG), or any mixture thereof, and
wherein the lipopolymer is a polyethylene glycol (PEG)- or polysarcosine-lipid conjugate or a PEG- or polysarcosine-lipid like conjugate, or any mixture thereof. An alternative aspect of the present invention relates to a lipid nanoparticle (LNP) composition comprising a cationic or cationically ionisable lipid or lipid-like material, a helper lipid, a lipopolymer, and monomycoloyl glycerol (MMG), wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-sn-glycero-3-phosphocholine (DOPC), 1,2-dioleoyl-*sn*-glycero-3-phospho-(1'-*rac*-glycerol) (DOPG), 1,2-dipalmitoyl-*sn*-glycero-3-phosphocholine (DPPC), 1,2-distearoyl-*sn-*glycero-3-phosphocholine (DSPC), 1,2-dipalmitoyl-*sn*-glycero-3-phosphoglycerol (DPPG), 1,2-dimyristoyl-*sn*-glycero-3-phosphocholine (DMPC), 1,2-diacyl-3-*O*-β-D-galactosyl-*sn*-glycerol (MGDG), 1-stearoyl-2-oleoyl-*sn*-glycero-3-phosphocholine (SOPC), 1,2-diacyl-3-*O*-(α-D-galactosyl1-6)-α-D-galactosyl-*sn-*glycerol (DGDG), and sulfoquinovosyldiacylglycerol (SQDG), or any mixture thereof, and
wherein the lipopolymer is a polyethylene glycol (PEG)- or polysarcosine-lipid conjugate or a PEG- or polysarcosine-lipid like conjugate, or any mixture thereof. The lipopolymers of the LNP compositions contribute to colloidal stabilisation of the LNPs. Often polyethylene glycol (PEG)-lipid conjugates or PEG-lipid like conjugates are used as the lipopolymer in LNP compositions. Thus, in an embodiment, the lipopolymer is a polyethylene glycol (PEG)-lipid conjugate or a PEG-lipid like conjugate. However, polysarcosine-lipid or polysarcosine-lipid like conjugates are being considered as a promising alternative to PEG-lipid or PEG-lipid like conjugates, since polysarcosine combines PEG-like properties, e.g., excellent solubility in water, protein resistance, low cellular toxicity and a non-immunogenic character, while being based on endogenous material. In addition, some people develop allergies to PEG, in which case it is particularly relevant to have an alternative to PEG-lipid or PEG-lipid like conjugates. Polysarcosine-functionalised LNPs have been developed in the literature and the described polysarcosine-based LNPs enable safe and efficient delivery of mRNA, thus signifying an excellent basis for the development of PEG-free RNA therapeutics. Hence, another embodiment relates to the LNP composition, wherein the lipopolymer is selected from the group consisting of 1,2-dimyristoyl-sn-glycero-3-phosphoethanolamine-N-[methoxy(polyethylene glycol)-2000] (DMPE-PEG₂₀₀₀), 1,2-dimyristoyl-*rac*-glycero-3-methoxypolyethylene glycol-2000 (DMG-PEG₂₀₀₀), 1,2-distearoyl-*sn*-glycero-3-phosphoethanolamine-N-[amino(polyethylene glycol)-2000] (DSPE-PEG₂₀₀₀), 2-[(polyethylene glycol)-2000]-N,N-ditetradecylacetamide (ALC-0159), N-(methylpolyoxyethylene oxycarbonyl)-1,2-dipalmitoyl-*sn*-glycero-3-phosphoethanolamine (DPPE-PEG), 1,2-distearoyl-rac-glycerol-3-methoxypolyethylene glycol (DSG-PEG), ceramide-PEG, 1,2-dipalmitoyl-*rac-*glycero-3-methylpolyoxyethylene (DPG-PEG), 1,2-dioleoyl-rac-glycerol, methoxypolyethylene glycol (DOG-PEG), 1,2-dioleoyl-*sn*-glycero-3-phosphoethanolamine-N-methylpolyoxyethylene (DOPE-PEG), N-tetradecyl polysarcosine25, N-hexadecyl polysarcosine25, N-octadecyl polysarcosine25, N-dodecyl polysarcosine25, N,N-ditetradecylamine-N-succinyl[methyl(polysarcosine)45], N,N-ditetradecylamine-N-succinyl[methyl(polysarcosine)35], and N,N-ditetradecyl-polysarcosine-25, or any mixture thereof.

In an embodiment, the cationic or cationically ionisable lipid or lipid-like material is 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), tetrakis(8-methylnonyl) 3,3',3",3‴-(((methylazanediyl)bis(propane-3,1-diyl))bis(azanetriyl))tetrapropionate (306Oi10), 9-heptadecanyl 8-{(2-hydroxyethyl)[6-oxo-6-(undecyloxy)hexyl]amino}octanoate (SM-102), 1,2-dioleoyl-3-trimethylamonniumpropane (DOTAP), dimethyldioctadecylammonium bromide (DDAB), 1,2-dioleoyl-3-dimethylammonium-chloride (DODAC), 1,2-di-*O-*octadecenyl-3-trimethylammonium propane (DOTMA), or [(4-hydroxybutyl)azanediyl]di(hexane-6,1-diyl) bis(2-hexyldecanoate) (ALC-0315), preferably C12-200 or SM-102.

In another embodiment, the helper lipid is 1,2-distearoyl-sn-glycero-3-phosphocholine (DSPC), 1-stearoyl-2-oleoyl-*sn*-glycero-3-phosphocholine (SOPC), or 1,2-dioleoyl-*sn*-glycero-3-phosphoethanolamine (DOPE), preferably DSPC or

### DOPE.

In yet another embodiment, the lipopolymer is 1,2-dimyristoyl-*sn*-glycero-3-phosphoethanolamine-N-[methoxy(polyethylene glycol)-2000] (DMPE-PEG₂₀₀₀), 1,2-distearoyl-*rac*-glycerol-3-methoxypolyethylene glycol (DSG-PEG), N-tetradecyl polysarcosine25, 1,2-distearoyl-sn-glycero-3-phosphoethanolamine-N-[amino(polyethylene glycol)-2000] (DSPE-PEG₂₀₀₀), or 1,2-dimyristoyl-*rac-*glycero-3-methoxypolyethylene glycol-2000 (DMG-PEG₂₀₀₀), preferably DMPE-PEG₂₀₀₀ or DMG-PEG₂₀₀₀.

In a preferred embodiment, the cationic or cationically ionisable lipid or lipid-like material is 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), the helper lipid is 1,2-dioleoyl-sn-glycero-3-phosphoethanolamine (DOPE), and the lipopolymer is 1,2-dimyristoyl-sn-glycero-3-phosphoethanolamine-N-[methoxy(polyethylene glycol)-2000] (DMPE-PEG₂₀₀₀).

### Defining the MMG

Cholesterol is one of the four components that LNPs usually comprise. However, the examples of the present invention demonstrate that cholesterol could successfully be replaced with monomycoloyl glycerol (MMG) analogues. MMG analogues possess immunopotentiating properties and can enhance immune responses thereby rendering the resulting LNPs more immunogenic. Thus, in an embodiment, the monomycoloyl glycerol (MMG) analogue is selected from the group consisting of MMG-1, MMG-2, MMG-3, MMG-4, MMG-5, MMG-6, and MMG-7, or any mixture thereof. In a preferred embodiment, the monomycoloyl glycerol (MMG) analogue is MMG-1, MMG-6, and/or MMG-7, preferably MMG-1.

In an embodiment, the monomycoloyl glycerol (MMG) analogue is added to a molar content in the range of 5 mol% to 70 mol%, such as 8 mol% to 60 mol%, such as 10 mol% to 50 mol% relative to the total molar content of the LNP composition. As shown in table 1, MMG-1 is added to a molar content in the range of 0 mol% to 48 mol% relative to the total molar content of the LNP composition.

### Defining the LNP composition - Cholesterol

An embodiment of the present invention relates to the lipid nanoparticle (LNP) composition, wherein said LNP further comprises cholesterol. In an embodiment, the cholesterol is added to a molar content in the range of 5 mol% to 70 mol%, such as 8 mol% to 60 mol%, such as 10 mol% to 50 mol% relative to the total molar content of the LNP composition.

As seen in table 1, cholesterol is gradually replaced with MMG-1 in the C12-200-based LNPs. Thus, in an embodiment, the ratio between the MMG analogue and cholesterol is in the range of 1:100 to 100:1, such as 1:4, such as 1:1, such as 4:1.

### Defining the content of the cationic or cationically ionisable lipid or lipid-like material, the helper lipid and the lipopolymer

The content of the cationic or cationically ionisable lipid or lipid-like material, the helper lipid and the lipopolymer may vary greatly based on what kind of lipids or lipid-like material or lipopolymer is used. Thus, in an embodiment, the cationic or cationically ionisable lipid or lipid-like material is added to a molar content in the range of 10 mol% to 60 mol%, such as 15 mol% to 55 mol%, such as 20 mol% to 50 mol%, such as 25 mol% to 45 mol%, preferably 30 mol% to 40 mol% relative to the total molar content of the LNP composition. In another embodiment of the present invention, the helper lipid is added to a molar content in the range of 5 mol% to 30 mol%, such as 8 mol% to 25 mol%, preferably 10 mol% to 20 mol% relative to the total molar content of the LNP composition. In a further embodiment, the lipopolymer is added to a molar content in the range of 0.5 mol% to 50 mol%, such as 0.8 mol% to 40 mol%, such as 1 mol% to 30 mol%, such as 2 mol% to 20 mol%, such as 5 mol% to 10 mol%, preferably 1 mol% to 2 mol% relative to the total molar content of the LNP composition.

### Defining the LNP composition - Nucleic acid

The LNP composition of the present invention could be used as a delivery system for several cargos, such as peptides or nucleic acids. Thus, in an embodiment, the LNP further comprises at least one nucleic acid. The nucleic acids may be a mixture of different nucleic acids, or it may be the same nucleic acid. Hence, in an embodiment, the at least one nucleic acid is a mixture of nucleic acids. Depending on the therapeutic purpose, several different nucleic acids may be relevant cargoes, such as mRNAs for use in an mRNA vaccine or siRNAs for use to knock down protein(s) of interest, or gRNA for use in the CRISPR/Cas9 technology to knock out a protein of interest. Hence, in an embodiment of the present invention, the at least one nucleic acid is selected from the group consisting of messenger RNA (mRNA), self-amplifying RNA, circular RNA (circRNA), plasmid DNA (pDNA), small interfering RNA (siRNA), single guide RNA (sgRNA), guide RNA (gRNA), long non-coding RNA (IncRNA), small activating RNA (saRNA), and splice-switching antisense oligonucleotide (ASO).

An siRNA could for instance be used to knock down the expression of tumour necrosis factor-a (TNF-a). Thus in an embodiment, the at least one nucleic acid is an siRNA, preferably targeting tumour necrosis factor-a (TNF-a) (SEQ ID NO.: 3, SEQ ID NO.: 4). In a preferred embodiment, the at least one nucleic acid is an RNA molecule or a mixture of RNA molecules. The nucleic acids used in the examples are RNAs, specifically mRNAs. Thus, in a more preferred embodiment, the at least one nucleic acid is an mRNA or a mixture of mRNAs.

The nucleic acids used as cargoes in these LNPs may be modified either by having chemically modified nucleosides or by having different bonds. These modifications can for example help stabilise the nucleic acids or make them more resistant to nucleases. Thus, in an embodiment, the at least one nucleic acid may comprise at least one chemically modified nucleoside, such as a pseudouridine, an N1-methyl pseudouridine, and a nucleoside with a 2'-O-methylation. In a further embodiment, the at least one nucleic acid may comprise phosphodiester bonds, phosphorothioate bonds, or a mixture thereof, preferably phosphodiester bonds.

### Vaccine composition

The LNP composition of the present invention is particularly useful as a vaccine composition, since the LNP composition has an improved immunogenicity as compared to LNP compositions without an MMG analogue as shown in example 4. Thus, an aspect of the present invention relates to a vaccine composition comprising the lipid nanoparticle (LNP) composition according to the present invention and at least one nucleic acid encoding an antigen. In an embodiment, the antigen is an antigen from a pathogen causing an infectious disease. In a further embodiment, the antigen is selected from the group consisting of corona virus antigens, such as SARS-CoV and MERS-CoV antigens, such as SARS-CoV2 spike protein (SEQ ID NO.: 5) or receptor binding domain (RBD), *Mycobacterium tuberculosis* antigens (SEQ ID NO.: 8-9), *Plasmodium falciparum* antigens (SEQ ID NO.: 10), respiratory syncytial virus (RSV) antigens (SEQ ID NO.: 11), Ebolavirus antigens, Marburg virus antigens, Lassa virus antigens, Nipah virus antigens, Zika virus antigens, Crimean-Congo haemorrhagic fever orthonairovirus antigens, human papilloma virus (HPV) antigens, and influenza antigens. As shown in the examples, the LNP composition can effectively deliver an mRNA cargo, and therefore the LNP composition is particularly useful in an mRNA vaccine. Hence, in an embodiment, the antigen is encoded by an mRNA.

### Use of the vaccine composition

An aspect relates to the vaccine composition according to the present invention for use in the prevention and/or treatment of an infectious disease. In an embodiment, the infectious disease is selected from the group consisting of tuberculosis, COVID-19, MERS-CoV infection, SARS-CoV infection, malaria, RSV infection, Ebola, Marburg virus infection, Lassa fever, Nipah virus infection, Zika virus infection, Crimean-Congo haemorrhagic fever, HPV infection, and influenza.

### Vaccine administration

The LNP composition was administered to mice using subcutaneous injection in example 3 and 4. However, as a vaccine composition said LNP composition according to the present invention and at least one nucleic acid encoding an antigen could be administered via other administration routes. Thus, in an embodiment, the vaccine composition is administered to a subject by intradermal, intraperitoneal, intravenous, intramuscular, or subcutaneous injection. Another embodiment relates to the vaccine composition for use according to the present invention, wherein the subject is a mammal, such as a human, a non-human primate, a calf, a pig, a horse, a sheep, a goat, a mink, a ferret, a hamster, a cat, a bird, or a dog. In a preferred embodiment, the subject is a human.

In an embodiment, the vaccine composition is administered as a single dose. However, vaccines are often given in a multidose regimen, e.g., the COVID-19 mRNA vaccines Spikevax^{®} and Comirnaty^{®}. Thus, in an embodiment, the vaccine composition is administered as at least two doses, such as at least three doses.

### Process for obtaining the lipid nanopartide (LNP) composition

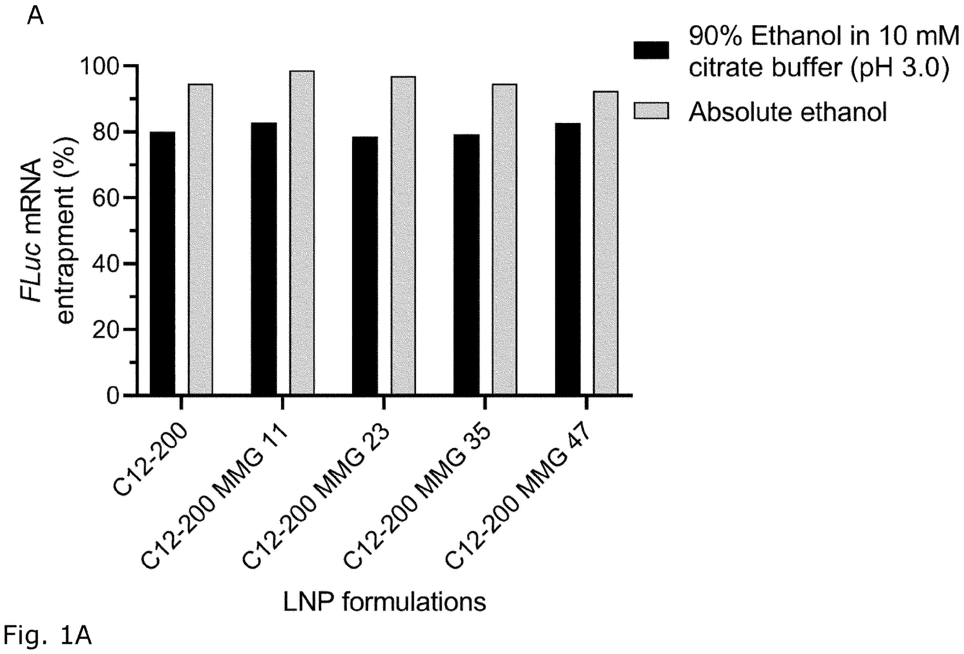

As shown in Figure 1A, the inventors found that the mRNA entrapment of *FLuc* mRNA (SEQ ID NO.: 1) loaded LNPs was increased when C12-200, MMG-1, DOPE, cholesterol, and DMPE-PEG₂₀₀₀ was dissolved in an organic phase consisting of absolute ethanol having a purity of at least 99.5%, preferably at least 99.8% ethanol as opposed to 90% ethanol with citrate buffer (10 mM, pH 3). Thus, an aspect relates to a process for obtaining the lipid nanoparticle (LNP) composition according to the invention, said process comprising the steps of:
a) Providing a cationic or cationically ionisable lipid or lipid-like material, a helper lipid, a lipopolymer, a monomycoloyl glycerol (MMG) analogue, and at least one nucleic acid;
b) Dissolving the cationic or cationically ionisable lipid or lipid-like material, the helper lipid, the lipopolymer, and the MMG analogue of step a) in an organic solvent comprising ethanol, preferably absolute ethanol with a purity close to 100%, thereby providing an organic phase;
c) Diluting the at least one nucleic acid of step a) in an aqueous solvent comprising a buffer with a pH within the range of 3 to 7.8, thereby providing an aqueous phase;
d) Mixing the organic phase of step b) with the aqueous phase of step c) to obtain lipid nanoparticles (LNPs) by nanoprecipitation;
e) Performing filtration, preferably tangential flow filtration or dialysis, of the LNPs of step d) to obtain an LNP composition;

wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-sn-glycero-3-phosphocholine (DOPC), 1,2-dioleoyl-sn-glycero-3-phospho-(1'-rac-glycerol) (DOPG), 1,2-dipalmitoyl-sn-glycero-3-phosphocholine (DPPC), 1,2-distearoyl-*sn-*glycero-3-phosphocholine (DSPC), 1,2-dipalmitoyl-sn-glycero-3-phosphoglycerol (DPPG), 1,2-dimyristoyl-sn-glycero-3-phosphocholine (DMPC), 1,2-diacyl-3-*O*-β-D-galactosyl-sn-glycerol (MGDG), 1-stearoyl-2-oleoyl-sn-glycero-3-phosphocholine (SOPC), 1,2-diacyl-3-*O*-(α-D-galactosyl1-6)-α-D-galactosyl-*sn-*glycerol (DGDG), and sulfoquinovosyldiacylglycerol (SQDG), or any mixture thereof,
wherein the lipopolymer is a polyethylene glycol (PEG)- or polysarcosine-lipid conjugate or a PEG- or polysarcosine-lipid like conjugate, or any mixture thereof, and
wherein the monomycoloyl glycerol (MMG) analogue is selected from the group consisting of MMG-1, MMG-2, MMG-3, MMG-4, MMG-5, MMG-6, and MMG-7, or any mixture thereof.

In an embodiment, said process further comprising the step:
f) Concentrating the lipid nanoparticle (LNP) composition using a method selected from the group consisting of filtration, centrifugation, vacuum-assisted centrifugation, or any mixture thereof, preferably filtration.

### Defining the cationic or cationically ionisable lipid or lipid-like material, the helper lipid, the lipopolymer and the monomycoloyl glycerol (MMG) analogue of step a)

An embodiment relates to the process according to the present invention, wherein the lipopolymer is selected from the group consisting of 1,2-dimyristoyl-*sn-*glycero-3-phosphoethanolamine-N-[methoxy(polyethylene glycol)-2000] (DMPE-PEG₂₀₀₀), 1,2-dimyristoyl-rac-glycero-3-methoxypolyethylene glycol-2000 (DMG-PEG₂₀₀₀), 1,2-distearoyl-sn-glycero-3-phosphoethanolamine-N-[amino(polyethylene glycol)-2000] (DSPE-PEG₂₀₀₀), 2-[(polyethylene glycol)-2000]-N,N-ditetradecylacetamide (ALC-0159), N-(methylpolyoxyethylene oxycarbonyl)-1,2-dipalmitoyl-sn-glycero-3-phosphoethanolamine (DPPE-PEG), 1,2-distearoyl-rac-glycerol-3-methoxypolyethylene glycol (DSG-PEG), ceramide-PEG, 1,2-dipalmitoyl-rac-glycero-3-methylpolyoxyethylene (DPG-PEG), 1,2-dioleoyl-rac-glycerol, methoxypolyethylene glycol (DOG-PEG), 1,2-dioleoyl-*sn-*glycero-3-phosphoethanolamine-N-methylpolyoxyethylene (DOPE-PEG), N-tetradecyl polysarcosine25, N-hexadecyl polysarcosine25, N-octadecyl polysarcosine25, N-dodecyl polysarcosine25, N,N-ditetradecylamine-N-succinyl[methyl(polysarcosine)45], N,N-ditetradecylamine-N-succinyl[methyl(polysarcosine)35], and N,N-ditetradecyl-polysarcosine-25, or any mixture thereof. In another embodiment, the cationic or cationically ionisable lipid or lipid-like material is 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), the helper lipid is 1,2-dioleoyl-sn-glycero-3-phosphoethanolamine (DOPE), and the lipopolymer is 1,2-dimyristoyl-sn-glycero-3-phosphoethanolamine-N-[methoxy(polyethylene glycol)-2000] (DMPE-PEG₂₀₀₀). In yet another embodiment, the monomycoloyl glycerol (MMG) analogue is MMG-1, MMG-6, and/or MMG-7, preferably MMG-1.

### Defining the nucleic acid of step a)

An embodiment relates to the process according to the present invention, wherein the at least one nucleic acid is selected from the group consisting of messenger RNA (mRNA), self-amplifying RNA (saRNA), circular RNA (circRNA), plasmid DNA (pDNA), small interfering RNA (siRNA), single guide RNA (sgRNA), guide RNA (gRNA), long non-coding RNA (IncRNA), small activating RNA (saRNA), and splice-switching antisense oligonucleotide (ASO), preferably the at least one nucleic acid is an mRNA.

### Defining the buffer of step c)

An embodiment relates to the process according to the present invention, wherein the pH of the buffer in step c) is in the range of 3.3 to 6, preferably 3.5 to 5, preferably the pH is 4.0. The pH value of the buffer might vary depending of what cationic or cationically ionisable lipid or lipid-like material is used. In an embodiment, the buffer is selected from the group consisting of citrate buffer, acetate buffer, Tris buffer, or HEPES buffer.

### Mixing by nanoprecipitation

Nanoprecipitation is a simple method used for encapsulation of both hydrophilic and hydrophobic drugs in nanoparticles. Nanoprecipitation can be performed using several different methods. Thus, in an embodiment, the mixing of step d) is performed using microfluidic mixing, pipette mixing, a T-mixer, or impingement jet mixers. In a preferred embodiment, the mixing is performed using microfluidic mixing. In another embodiment, the flow rate ratio of the aqueous phase to the organic phase during the microfluidic mixing is in the range of 1:1 to 10: 1, preferably 3: 1. In yet another embodiment, the total flow rate during microfluidic mixing is selected from the range of 1.0 mL/min to 20 mL/min, preferably the flow rate is 12 mL/min.

### Defining the filtration of step e)

In an embodiment, the filtration of the lipid nanoparticles (LNPs) in step e) is performed against phosphate-buffered saline or Tris buffer, preferably Tris buffer at a pH of 7.4.

### Product by process

An aspect relates to a lipid nanoparticle (LNP) composition obtained using the process of the present invention.

It should be noted that embodiments and features described in the context of one of the aspects of the present invention also apply to the other aspects of the invention. All patent and non-patent references cited in the present application, are hereby incorporated by reference in their entirety.

The invention will now be described in further details in the following non-limiting examples.

### Examples

### Example 1 - Materials and methods

### Materials

CleanCap^{®} enhanced green fluorescent protein *(eGFP), FLuc* (SEQ ID NO.: 1), and *OVA* mRNA (SEQ ID NO.: 2) fully substituted with 5-methoxyuridine (1 mg/mL in 1 mM sodium citrate buffer, pH 6.4) were acquired from TriLink Biotechnologies (San Diego, CA, USA). C12-200 was synthesised, purified, and characterised as previously reported (Love, K.T *et al.* 2010). MMG-1 was purchased from Clausson Kaas (Farum, Denmark). 1,2-distearoyl-*sn*-glycero-3-phosphocholine (DSPC) and 1,2-dioleoyl-*sn*-glycero-3- phosphoethanolamine (DOPE) were acquired from Avanti^{®} Polar Lipids (Alabaster, AL, USA). Cholesterol, 1,2-dimyristoyl-*rac-*glycero-3-methoxypolyethylene glycol-2000 (DMG-PEG) and 1,2-dimyristoyl-*sn-*glycero-3-phosphoethanolamine-N-[methoxy(polyethylene glycol)-2000] (DMPE-PEG) were obtained from Sigma-Aldrich (St. Louis, MO, USA). SM-102 was acquired from MedChemExpress (Copenhagen, Denmark). EndoFit^{™} OVA was obtained from Invivogen (Toulouse, France). Triton^{™} X-100 was purchased from Sigma-Aldrich. Quant-iT^{™} RiboGreen^{®} RNA Reagent and Tris-EDTA buffer (10 mM Tris, 1 mM EDTA, pH 8.0) (TE buffer) were acquired from Molecular Probes, Invitrogen (Paisley, UK). RNase-free water was used throughout the studies. All other chemicals and reagents were of analytical grade, and were acquired from commercial suppliers.

### Preparation of mRNA-loaded lipid nanoparticles

C12-200 LNPs were prepared by microfluidic mixing using the NanoAssemblr^{®} Ignite^{™} microfluidic mixer system (Precision Nanosystems Inc., Vancouver, Canada), which uses a toroidal micromixer chip. The aqueous phase consisted of mRNA *(FLuc)* dissolved in citrate buffer (10 mM, pH = 3). C12-200 or SM-102, MMG-1, DOPE, cholesterol, and DMPE-PEG were dissolved in an organic phase consisting of absolute ethanol having a purity of 99.99%, and the MMG-1:cholesterol molar ratio was varied systematically **(Table 1).** For ease of nomenclature, MMG-1 modified C12-200 LNPs are referred to as C12-200 MMG 11 (11.88 mol % MMG-1), C12-200 MMG 23 (23.25 mol % MMG-1), C12-200 MMG 35 (35.63 mol % MMG-1), and C12-200 MMG 47 (47.5 mol % MMG-1), respectively. As a control, two C12-200 LNP formulations without MMG-1 were prepared: one with cholesterol (called C12-200) and another without cholesterol (called C12-200 DOPE DMPE-PEG₂₀₀₀). For comparison, LNPs containing 0 mol % or 37.5 mol % MMG-1 were also prepared, where SM-102 was used as the ionisable lipid **(Table 1).** Said SM-102 LNPs were prepared using DSPC as the helper lipid, whereas "SM-102 DOPE MMG 38" comprised DOPE as the helper lipid. LNPs were prepared by injecting the organic phase into the central inlet and the aqueous phase into the right inlet of the micromixer chip. The mRNA and lipid solutions were mixed at a flow rate ratio (FRR) of aqueous to organic phase of 3:1 (v/v). A total flow rate (TFR) of 10 mL/min was employed. The start waste (0.25 mL), end waste (0.10 mL), and dead volume of the syringes, and possible losses during loading of the syringes into the microfluidics mixing system, were considered in the calculations of the formulation volumes. The final formulation volume was 1.25 mL. The C12-200 and MMG-1-based LNPs used for immunogenicity studies were prepared in a similar way (FFR = 3:1, TFR = 10 mL/min, end waste = 1.5 mL), and were loaded with 80 µg *OVA* mRNA in a final formulation volume of 4.5 mL. The LNPs used to immunise the control group (Ctrl) were loaded with 50 µg *eGFP* mRNA for priming and *FLuc* mRNA for boost immunisation, respectively. The formulations were purified by dialysis against phosphate-buffered saline (PBS) using 10,000 MWCO dialysis cassettes (Slide-A-Lyzer^{®}, Thermo Fisher Scientific, Waltham, MA, USA) for 2.5 h under magnetic stirring to exchange the solvent and neutralise the pH. The formulations were subsequently upconcentrated in PBS using a 100 kDa MWCO 4 mL centrifugal filter (Amicon^{®}, Merck KGaA, Darmstadt, Germany) by centrifugation for 4 min at 1500 × g at 20 °C to a final lipid content of approximately 1.5 mg/mL.

**Table 1: Theoretical molar composition of C12-200/SM-102- and MMG-1-modified C12-200/SM-102 lipid nanoparticles (LNPs)**

| **Formulations** | **Composition (mol %)** | | | | |
|---|---|---|---|---|---|
| | C12-200 | DOPE | Cholester ol | MMG-1 | DMPE-PEG₂₀₀₀ |
| C12-200 | 35 | 16 | 47.5 | 0 | 1.5 |
| C12-200 MMG 11 | 35 | 16 | 35.63 | 11.88 | 1.5 |
| C12-200 MMG 23 | 35 | 16 | 23.75 | 23.75 | 1.5 |
| C12-200 MMG 35 | 35 | 16 | 11.88 | 35.63 | 1.5 |
| C12-200 MMG 47 | 35 | 16 | 0 | 47.5 | 1.5 |
| C12-200 DOPE DMPE-PEG2000 | 66.65 | 30.48 | 0 | 0 | 2.87 |

| | SM-102 | DSPC | Cholester ol | MMG-1 | DMG-PEG₂₀₀₀ |
|---|---|---|---|---|---|
| SM-102 | 50 | 10 | 38.5 | 0 | 1.5 |
| SM-102 DSPC MMG 37 | 50 | 10 | 0 | 37.5 | 2.5 |
| | SM-102 | DOPE | Cholester ol | MMG-1 | DMG-PEG₂₀₀₀ |
| SM-102 DOPE MMG 38 | 50 | 10 | 0 | 38.5 | 1.5 |

### Statistical analysis

Graphing and statistical analysis were performed using GraphPad Prism v9 (Graphpad Software Inc, La Jolla, CA, USA). The *in vivo* imaging data was analysed by one-way analysis of variance (ANOVA) at a 0.05 significance level, and pair-wise comparison was performed using Tukey's post-test. Immune responses were compared between the groups by one-way ANOVA (cytokine-producing T cells, T_{FH} and GC B cells, antibodies) or two-way ANOVA (polyfunctional T cells) at a 0.05 significance level, and pair-wise comparison was performed using Tukey's or Dunnett's post-test, respectively. A value of p < 0.05 was considered statistically significant.

### Example 2 - Cholesterol can be replaced with MMG-1 in C12-200 LNPs and in SM-102 LNPs

### Aim of study

The aim of this study was to test whether step-wise replacement of cholesterol with MMG-1 would affect the physicochemical properties, the mRNA entrapment, and the morphology of the resulting LNPs.

### Materials and methods

### Physicochemical characterisation

The LNP formulations were characterised with respect to average intensity-weighted hydrodynamic diameter (z-average) and polydispersity index (PDI). The z-average and PDI were determined at 25 °C by dynamic light scattering using the photon correlation spectroscopy technique with a ZetaSizer Nano SZ (Malvern Instruments, Worcestershire, UK) equipped with a 633 nm laser at a 173° detection angle. The Zetasizer 7.11 software (Malvern Instruments) was used for data acquisition and analysis. Prior to analysis, 60 µL formulation was diluted with 440 µL PBS in a 1 mL polystyrene cuvette. The measurements were performed in triplicate with an equilibration time of 60 s. The mRNA entrapment efficiency was quantified using the Quant-iT^{™} RiboGreen^{®} assay, essentially as described previously (Lokras, A. *et al.* 2022).

### Morphology of MMG-1-modified LNPs

Morphological analysis of *FLuc* mRNA-loaded LNP formulations C12-200, C12-200 MMG 23, and C12-200 MMG 47 was carried out by cryogenic transmission electron microscopy (cryo-TEM) using a Tecnai G2 20 TWIN transmission electron microscope (Field Electron and Ion Company, Hillsboro, Oregon, USA). Samples were prepared by vitrification (rapid cooling) and blotted onto a Pelco Lacey carbon filmed grid using a Vitrobot^{™} Mark IV (Field Electron and Ion Company). Excess liquid was removed with a filter paper forming a thin film of approximately 10-500 nm. The samples where then immediately plunged into liquid ethane at - 180 °C and transported in a cryo-holder that was connected to the electron microscope. The samples were kept below -180 °C throughout the experiment. Analysis was performed in the bright field mode at an accelerating voltage of 120 mV. Digital pictures were recorded using a Gatan Imaging Filter 100 CCD camera (Gatan, Pleasanton, CA, USA).

### Results

*FLuc* and *OVA* mRNA-loaded C12-200 LNPs and modified C12-200 LNPs with different molar contents of MMG-1 were prepared by microfluidic mixing using a NanoAssemblr^{®} Ignite^{™} microfluidics mixing system. The *FLuc* mRNA-loaded SM-102 LNPs were also prepared using the NanoAssemblr^{®} Ignite^{™} microfluidics mixing system. *FLuc* mRNA-loaded LNPs used for imaging had an average hydrodynamic diameter below 200 nm for all LNP formulations, and the average PDI values were below 0.25 **(Table 2),** indicating that the formulations were monodisperse. The SM-102 LNPs had in general a lower average hydrodynamic diameter than the C12-200 LNPs, however, the incorporation of MMG-1 did not seem to affect this difference. The *OVA* mRNA-loaded LNPs used for *in vivo* immunogenicity studies, prepared with or without MMG-1, displayed an average hydrodynamic diameter below 140 nm, while the average PDI values were below 0.16 **(Table 2).** The *eGFP* or *FLuc* mRNA-loaded LNPs used as control groups in the immunisation experiment displayed average hydrodynamic diameters of 106 and 115 nm and PDI values of 0.149 and 0.075, respectively. The entrapment efficiency (%) of LNPs loaded with *OVA* mRNA (used for immunogenicity) was slightly lower (average 88%) than the entrapment efficiency of *FLuc* mRNA-loaded LNPs (average 94%) (used for imaging) irrespective of the MMG-1 modification **(Table 2).** The mRNA entrapment efficiency of control formulations used for the *in vivo* immunogenicity study was 93 % for *eGFP* mRNA and 95 % for *FLuc* mRNA. No significant differences were observed in the entrapment efficiencies for the MMG-1-modified or non-modified C12-200 LNPs used for imaging or *in vivo* immunogenicity studies. Interestingly, the inventors found that dissolving C12-200, MMG-1, DOPE, cholesterol, and DMPE-PEG₂₀₀₀ in an organic phase consisting of absolute ethanol as opposed to 90% ethanol with citrate buffer (10 mM, pH 3) increased the mRNA entrapment of *FLuc* mRNA loaded LNPs from approximately 80% to approximately 90-96% **(****Figure 1A****).** Thus, the LNPs formulated using absolute ethanol was used in the subsequent experiments.

**Table 2: LNP characteristics. Data represent mean values of two independent formulations.**

| **Formulations** | **Z-average (nm)** | **Polydispersity index (PDI)** | **mRNA entrapment (%)** |
|---|---|---|---|
| LNPs with *FLuc* mRNA | | | |
| C12-200 | 115 | 0.075 | 94.6 |
| C12-200 MMG 11 | 194 | 0.090 | 98.7 |
| C12-200 MMG 23 | 198 | 0.160 | 96.9 |
| C12-200 MMG 35 | 181 | 0.080 | 94.6 |
| C12-200 MMG 47 | 137 | 0.137 | 92.4 |
| C12-200 DOPE DMPE-PEG2000 | 331 | 0.210 | 82.0 |
| SM-102 | 85 | 0.112 | 98.8 |
| SM-102 DSPC MMG 37 | 73 | 0.137 | 95.8 |
| SM-102 DOPE MMG 37 | 115 | 0.157 | 98.3 |

| LNPs with *OVA* mRNA | | | |
|---|---|---|---|
| C12-200 | 136 | 0.155 | 92.6 |
| C12-200 MMG 11 | 105 | 0.083 | 88.4 |
| C12-200 MMG 23 | 128 | 0.073 | 85.9 |
| C12-200 MMG 35 | 117 | 0.091 | 88.0 |
| C12-200 MMG 47 | 121 | 0.100 | 89.0 |

| LNPs with *eGFP* mRNA | | | |
|---|---|---|---|
| C12-200 | 106 | 0.149 | 92.5 |

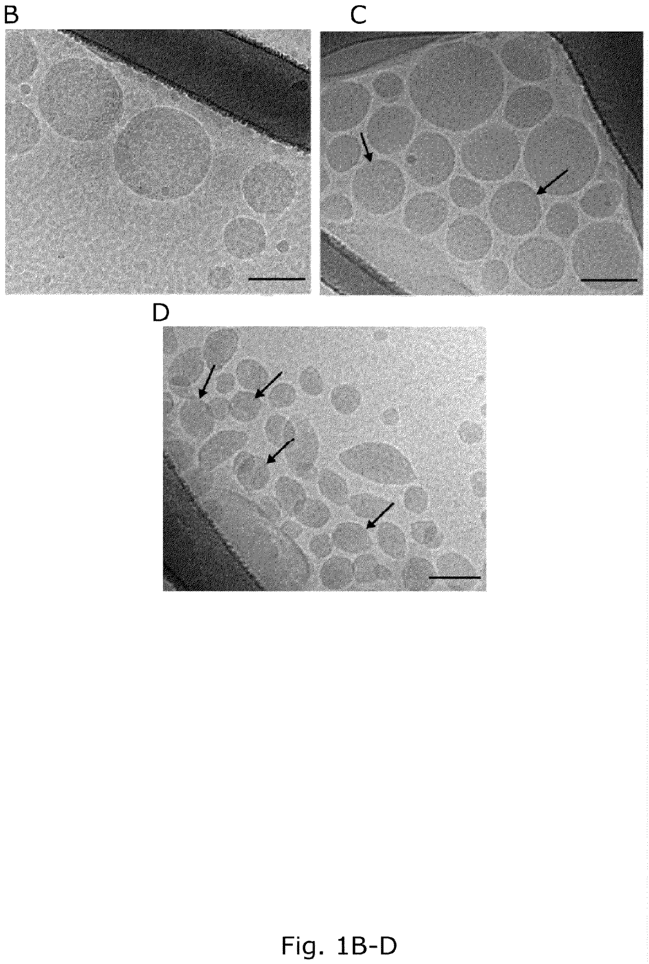

For the morphological analysis of LNPs by cryo-TEM, *FLuc* mRNA-loaded C12-200, C12-200 MMG 23, and C12-200 MMG 47 were selected **(**Figure 1B-D). All LNPs were approximately 100-200 nm in diameter, which is consistent with their average hydrodynamic diameter. C12-200 LNPs displayed a spherical morphology with a uniform curvature **(****Figure 1B****),** while C12-200 MMG 23 LNPs were less spherical and had a more edgy surface **(****Figure 1C****).** On the other hand, C12-200 MMG 47 LNPs displayed a characteristic faceted morphology **(****Figure 1D****).**

### Conclusion

There were no significant differences between the average hydrodynamic diameters (z-average), PDI, and mRNA entrapment efficiencies of MMG-1-modified C12-200 LNPs as compared to said parameters in the unmodified C12-200 LNPs. However, the morphology of the LNPs changed as more cholesterol was replaced by MMG-1, i.e. the morphology of C12-200 LNPs was spherical with a uniform curvature, became less spherical and had a more edgy surface in C12-200 MMG 23, and became faceted in C12-200 MMG 47. In addition, MMG-1-modified LNPs could be formulated using SM-102, suggesting that other cationic or cationically ionisable lipid or lipid-like materials can be used.

### Example 3 - Replacing cholesterol with MMG-1: Effect on the biodistribution of FLuc-loaded C12-200 LNPs and SM-102 LNPs

### Aim of study

The aim of this study was to test whether replacing cholesterol with MMG-1 in C12-200 and SM-102 LNPs affected the biodistribution and duration of protein production mediated by said C12-200 and SM-102 LNPs loaded with FLuc mRNA.

### Materials and methods

### Bioluminescence imaging

Female 8-10 weeks old BALB/cOlaHsd mice were acquired (ENVIGO, Horst, Netherlands) and acclimatised for one week before imaging. Food and water were supplied *ad libitum.* All experimental work was approved by the Danish National Experiment Inspectorate under permit 2022-15-0201-01221, and was performed in accordance with the European Community directive 86/609 for the care and use of laboratory animals. Mice were injected subcutaneously (s.c.) at the base of the tail with 4 µg *FLuc* mRNA loaded into either C12-200 and MMG-1-modified C12-200 LNPs or SM-102 and MMG-1-modified SM-102 LNPs, using a dose volume of 100 µL PBS (pH 7.4). After 6, 24, 48, 72, and 96 h post-administration, the mice were injected intraperitoneally (i.p.) with 150 mg luciferin/kg body weight of XenoLight D-luciferin potassium salt bioluminescent substrate (PerkinElmer, Waltham, MA, USA) and rested for 15 min. The mice were then anesthetised using isoflurane and imaged by using an In Vivo Imaging System (IVIS) Lumina XRMS (PerkinElmer). The bioluminescence emission was measured, and the intensity of the emitted light as radiance (photons/cm²/steradian) and total flux (p/s) was quantified by using the Living Image^{®} Software v4.7.4 (PerkinElmer).

### Results

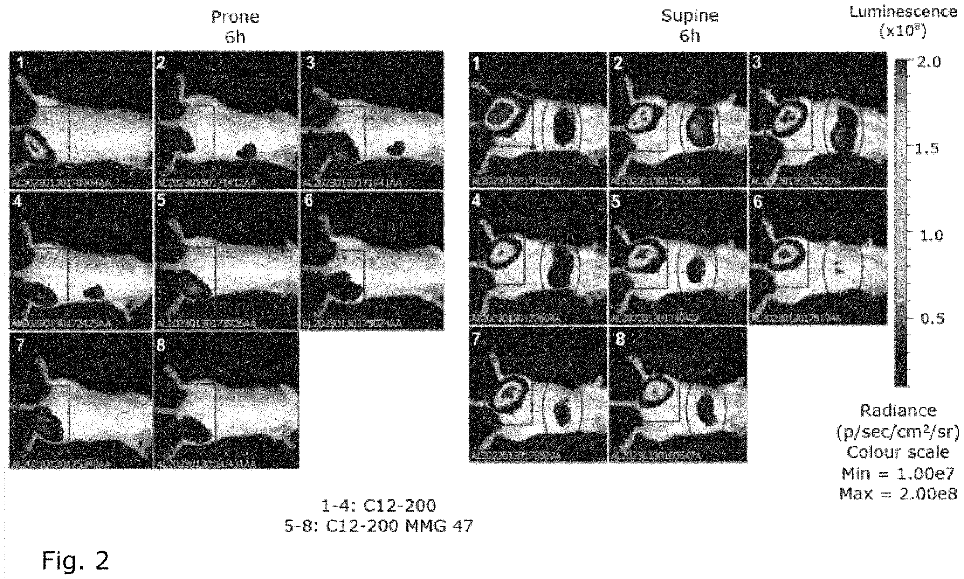

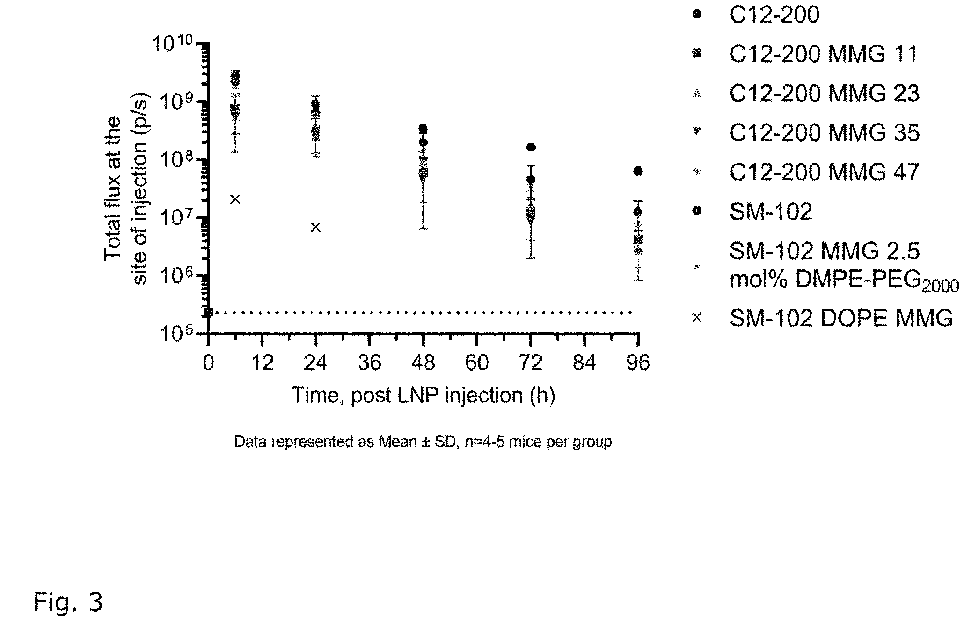

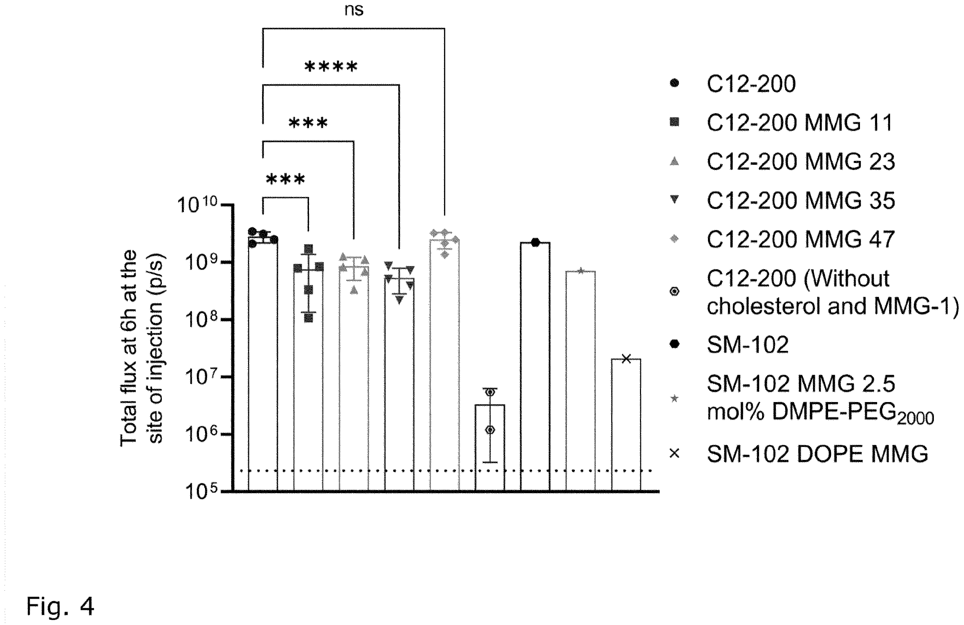

To examine the biodistribution and duration of protein production mediated by mRNA-loaded LNPs *in vivo,* unmodified and MMG-1-modified C12-200 and SM-102 LNPs loaded with *FLuc* mRNA (Table 2), were administered s.c. to mice at the base of the tail at the dose of 4 µg mRNA, and the spatiotemporal *FLuc* expression was imaged and quantified at 6 h, 24 h, 48, 72, and 96 h **(****Figure 2-4****).** Only the images captured 6 h after LNP administration are shown in figure 2. Whole-body images of mice captured from the supine position demonstrated strong bioluminescence signals at the site of injection (SOI), i.e., the base of the tail, for all LNP formulations 6 h after dosing **(****Figure 2****).** The bioluminescence images from the supine position showed for some of the mice translocation of the MMG-1-modified C12-200 LNPs to the liver, as compared to unmodified C12-200 LNPs, which largely remained at the SOI **(****Figure 2****).** No significant difference in the bioluminescence signal was observed at any time point between C12-200 and C12-200 MMG 47 LNP, with the signal being at least 10 times above background at 96 h post injection **(****Figure 3****).** Quantification of *FLuc* expression at the SOI (supine) showed a significantly lower protein production at 6 h after dosing for mice injected with the C12-200 MMG 11, 23, and 35 LNP formulations, as compared to the C12-200 and C12-200 MMG 47 LNP formulations (***p < 0.001 ****p < 0.0001) **(****Figure 4****).** In addition, high FLuc expression was observed for mice dosed with SM-102 LNPs, and the expression levels were comparable to the levels measured for mice dosed with the SM-102 MMG 37 formulation. Interestingly, LNPs formulated without cholesterol or MMG-1 did not mediate FLuc expression **(****Figure 4****).**

### Conclusion

A strong bioluminescence signal intensity at the SOI 6 h post-administration in mice confirms that the MMG-1-modified C12-200/SM-102 LNPs are capable of mediating *in vivo* transfection and intracellular delivery of mRNA as efficiently as the non-modified C12-200 LNPs.

### Example 4 - Replacing cholesterol with MMG: Effect on the immunogenicity

### Aim of study

The aim of this study was to test whether replacing cholesterol with MMG-1 in the C12-200 LNPs affected the immunogenicity of said LNPs loaded with OVA mRNA.

### Materials and methods

### Immunisations

Six-week old female C57BL/6 mice (ENVIGO) were acquired and acclimatised for one week before experimental manipulation. Animals had access to food and water *ad libitum.* All experimental work was approved by the Danish National Experiment Inspectorate under permit 2016-15-0201-01026. The studies were performed in accordance with the European Community directive 86/609 for the care and use of laboratory animals. Mice were assigned to six groups of six individuals. All mice were immunised twice by s.c. administration at the base of tail at an interval of 2 weeks using a dose volume of 200 µL PBS (pH 7.4). The control groups (Ctrl) of mice were primed with saline (n = 3) and 10 µg *eGFP* mRNA-loaded C12-200 LNPs (n = 3), respectively, and boosted with saline (n = 3) and 10 µg *Fluc* mRNA-C12-200 LNPs (n = 3), respectively. Mice in the C12-200 group were immunised twice with 10 µg *OVA* mRNA-loaded C12-200 LNPs (C12-200, n = 6). Mice in the four last groups were immunised twice with 10 µg *OVA* mRNA-loaded C12-200 LNPs containing 11 (C12-200 MMG 11, n = 6), 23 (C12-200 MMG 23, n = 6), 35 (C12-200 MMG 35, n = 6), and 47 (C12-200 MMG 47, n = 6) mol% of MMG-1, respectively. The final doses of C12-200/DOPE/cholesterol/MMG-1/DMPE-PEG were 267/80/123/0/27, 267/80/123/0/27, 267/80/92/45/27, 267/80/61/91/27, 267/80/31/136/27, and 267/80/0/182/27 µg/mice/immunisation, for the groups Ctrl, C12-200, C12-200 MMG 11, C12-200 MMG 23, C12-200 MMG 35, and C12-200 MMG 47, respectively.

### Sample collection and cell preparation

At 4 weeks of the study, i.e., two weeks after the booster immunisation, animals were euthanised. Blood was collected by cardiac puncture, and serum was separated by spontaneous clotting at room temperature (RT) and extracted by centrifugation (2000 × g, for 10 min) using a Heraeus Multifuge 3SR+ (Thermo Fischer Scientific). Serum was stored at -20 °C until antibody detection. The spleen and LNs draining the s.c. administration site, i.e., the inguinal LNs, were aseptically harvested from the euthanised mice. The spleen and LNs were homogenised using a 70 µm nylon mesh cell-strainer (Falcon, Durham, NC, USA), and washed twice with PBS to obtain single-cell suspensions. Spleen and LN cells were then grown in 96-well microtiter plates (Nunc, Roskilde, Denmark) containing 2 × 10⁵ cells per well for cytokine assays, or 1 × 10⁶ cells per well for flow cytometry, in 100 µL RPMI-1640 (Sigma-Aldrich) supplemented with 5 × 10⁻⁵ M 2-mercaptoethanol (Gibco Thermo Fisher), 1% (v/v) sodium pyruvate (Sigma-Aldrich), 1% (v/v) penicillin-streptomycin (Gibco Thermo Fisher), 1% HEPES (Gibco Thermo Fisher), and 10% (v/v) FCS (Gibco Thermo Fisher).

### Antibody detection

Antibodies were detected in serum samples using ELISA. Briefly, Maxisorp^{™} plates (Nunc) were coated with 1 µg/mL OVA solution in carbonate-bicarbonate buffer (pH = 9.6). Serum samples were 5-fold serially diluted 11 times from a 1:5 dilution with bicarbonate buffer. IgG, IgG1, and IgG2c specific for OVA were detected with horseradish peroxidase-conjugated secondary antibodies, i.e., rabbit anti-mouse IgG (ThermoFisher; diluted 1:2,500), goat anti-mouse IgG1 (Southern Biotech, Birmingham, AL, USA; diluted 1:20,000), and goat anti-mouse IgG2c (Southern Biotech; diluted 1:5,000), respectively. 3,3',5,5'-tetramethylbenzidine Plus2 (Kem-En-Tec, Taastrup, Denmark) was used as substrate. The enzymatic reaction was stopped by addition of 0.2 M H₂SO₄, and absorbance was read at a wavelength of 450 nm using a FLUOstar optima plate reader (BMG Labtech, Ortenberg, Germany). Non-linear regression analysis was performed on serum O.D. values to calculate the ELISA mid-point titers, i.e., EC₅₀ as previously described (Thakur, A. *et al.* 2018).

### Flow cytometry

Splenocytes and LN cells were stimulated with OVA (SEQ ID NO.: 2) (5 µg/mL), OVA₂₅₇₋₂₆₄ (SIINFEKL) (SEQ ID NO.: 6) and OVA₃₂₃₋₃₃₉ peptide (ISQAVHAAHAEINEAGR) (SEQ ID NO.: 7) (both 5 µg/mL, AnaSpec, Fremont, CA, USA), respectively, supplemented with anti-CD28 (37.51) and anti-CD49d (9C10) co-stimuli (both 1 µg/mL, BD Biosciences), at 37°C, 5% CO₂ for 6 h, with brefeldin A (10 µg/mL, Sigma-Aldrich) and monensin/Golgi-stop (0.7 µL/mL, BD Bioscience) added during the last 5 h of incubation. Medium alone and concanavalin A (5 µg/mL, Sigma Aldrich) served as negative and positive controls, respectively. For the detection of polyfunctional epitope-specific CD4⁺ T cells, a previously described protocol was used that combines MHC-II tetramer and intracellular cytokine staining (ICS) (Pastore, G. *et al.* 2019). Based on this protocol, the detection of polyfunctional epitope-specific CD8⁺ T cells was optimised by combining MHC-I pentamer staining with ICS. Following overnight storage at 4 °C, the spleen and LN cells were labelled with Fixable Viability Stain (FVS) 510 (BD Biosciences, 1:1,000, 100 µL/well) for 20 min at 4 °C in the dark, and washed twice with PBS. Cells were fixed and permeabilised for 20 min at 4 °C with BD Cytofix/Cytoperm (BD Biosciences). Samples were then blocked for 30 min at 4 °C in Fc-blocking solution (5 µg/mL CD16/CD32 mAb, BD Bioscience), and stained for 1 h at RT with PE-conjugated H-2Kb-SIINFEKL (OVA₂₅₇₋₂₆₄) (MHC-I) pentamer (diluted 1:8, ProImmune, Oxford, UK) and PE-conjugated I-A^{b}-ISQAVHAAHAEINEAGR (OVA₃₂₃₋₃₃₉) (MHC-II) tetramer (diluted 1:8, ProImmune), respectively, diluted in perm/wash buffer. In the final 20 min of the tetramer incubation period, the following mix of fluorescent antibodies was added: anti-CD4-BUV395 (RM4-5; BD Biosciences), anti-CD8-BUV737 (53-6.7; BD Biosciences), anti-CD44-FITC (IM7; BD Biosciences), anti-CD62L-PE-CF594 (MEL-14; BD Biosciences), anti-IFN-γ-PE-Cy7 (XMG1.2; eBioscience), anti-TNF-α-BV711 (MP6-XT22; BD Biosciences), anti-IL-2-APC (JES6-5H4; eBioscience), anti-IL-4-BV421 (11B11; BD Biosciences), and anti-IL-17-PerCP-Cy5.5 (eBio17B7; eBioscience). For the detection of T_{FH} and GC B cells, LN cells were stained with anti-CD3-BV711 (145-2C11; BD Biosciences), anti-CD4-BUV395 (RM4-5; BD Biosciences), anti-B220-BV480 (RA3-6B2; BD Biosciences), anti-CD95-AF647 (Jo2; BD Biosciences), anti-CXCR5-PE (2G8; BD Biosciences), anti- GL7-FITC (GL7; BD Biosciences), and anti-PD-1-BV421 (J43; BD Biosciences). Dead cells were excluded by using the fixable viability dye FVS780 (BD Biosciences). All cells were twice washed, resuspended in FACS buffer, and analysed using an LSRFortessa flow cytometer (BD Biosciences). Gates for the surface markers are based on fluorescence-minus-one controls. The gating strategy used to identify distinct cell populations in the spleen and the draining LNs is based on previous reports (Vono, M. *et al.* (2019), Christensen, D. *et al.* (2017), Thakur, A. *et al.* (2018)). All flow cytometric data analyses were performed using the FlowJo software v10 (Tree Star, Ashland, OR, USA).

### Results

### Unmodified and MMG-1-modified, OVA mRNA-loaded C12-200 LNPs induce high CD8⁺ T cell responses in the lymph nodes draining the s.c. site of injection

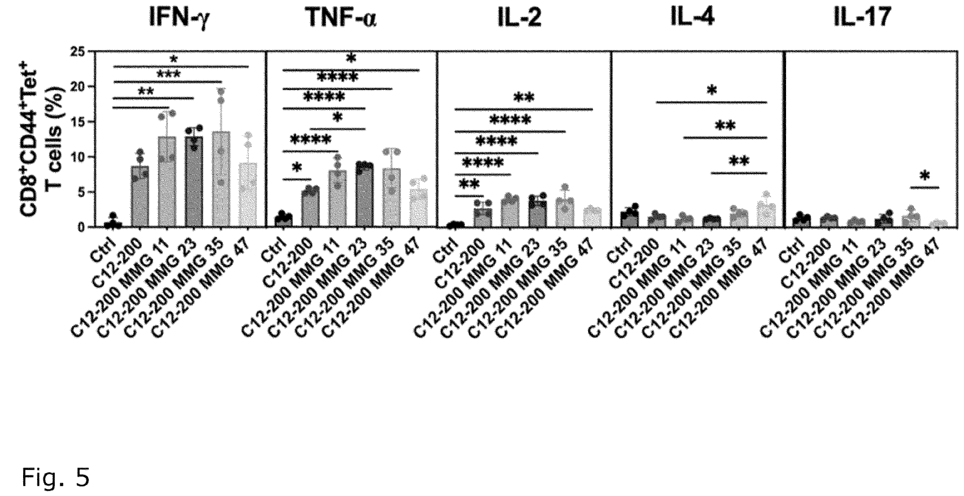

To study the effect of replacing cholesterol with MMG-1 on immunogenicity, the inventors measured the immunogenicity *of OVA* mRNA-loaded LNPs in mice following prime and boost immunisation s.c. at 2 weeks interval. Immune responses were assessed by *ex vivo* re-stimulation of isolated LN cells with antigen, followed by intracellular cytokine staining (ICS) in combination with tetramer staining and quantification of phenotypic marker expression **(****Figure 5****).** The functionality of the antigen-specific CD4⁺CD44⁺ and CD8⁺CD44⁺ T cells was determined with respect to their expression of IFN-γ, TNF-a, IL-2, IL-4, and IL-17 by combinatorial Boolean gating analysis. In general, immunisation with *OVA* mRNA-loaded unmodified and MMG-1-modified C12-200 LNPs resulted in very high percentages of OVA₂₅₇₋₂₆₄-specific MHC-I (SIINFEKL) (SEQ ID NO.: 6) Tet⁺CD8⁺CD44⁺ T cells that produce IFN-γ in the draining LNs. In addition, immunisation with *OVA* mRNA-loaded MMG-1-modified C12-200 LNPs resulted in significantly higher percentage of OVA₂₅₇₋₂₆₄-specific MHC-I (SIINFEKL) (SEQ ID NO.: 6) Tet⁺CD8⁺CD44⁺ T cells that produced TNF-α (*p < 0.05 for *OVA* mRNA-loaded C12-200 MMG 23) as compared to immunisation with *OVA* mRNA-loaded C12-200 LNPs **(****Figure 5****).** A statistically significantly higher percentage of OVA₂₅₇₋₂₆₄-specific CD8⁺CD44⁺ T cells that co-produced IFN-γ and TNF-a was measured for mice vaccinated with *OVA* mRNA-loaded C12-200 MMG 11 (*p < 0.05) and C12-200 MMG 35 LNPs (****p < 0.0001), respectively, than for mice vaccinated with *OVA* mRNA-loaded C12-200 LNPs. No other significant differences between the LNP formulations were observed for the polyfunctional CD8⁺CD44⁺ T cell responses (data not shown).

### Replacing cholesterol with MMG-1 does not affect the CD4⁺ T cell responses induced by OVA mRNA-loaded C12-200 LNPs in the draining lymph nodes

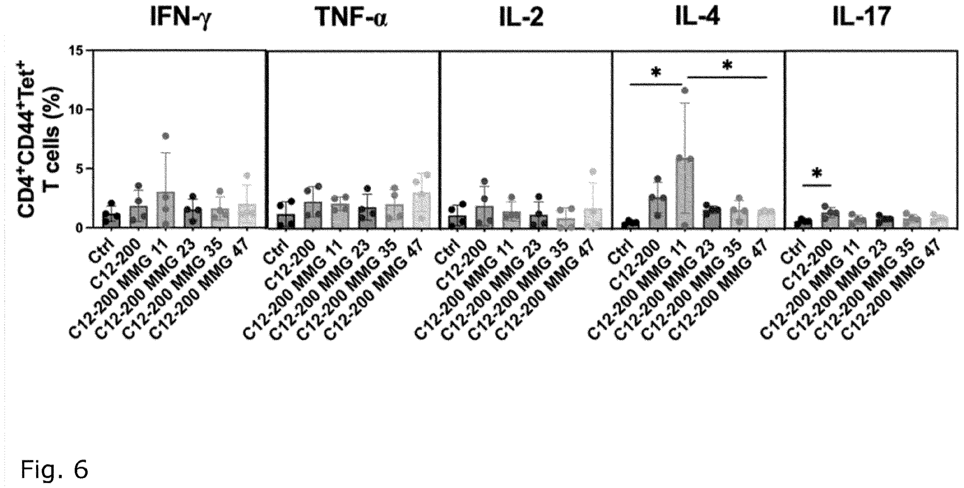

The inventors then measured OVA₃₂₃₋₃₃₉-specific CD4⁺CD44⁺ T cells in the draining LNs. There were largely no statistically significant differences between the different groups with respect to percentages of cytokine-producing, OVA₃₂₃₋₃₃₉-specific MHC-II (ISQAVHAAHAEINEAGR) (SEQ ID NO.: 7) Tet⁺CD4⁺CD44⁺ T cells **(****Figure 6****).** OVA₃₂₃₋₃₃₉-specific CD4⁺CD44⁺ T cells that produced IL-4 were only significantly higher for *OVA* mRNA-loaded C12-200 MMG 11 than *OVA* mRNA-loaded C12-200 MMG 47.

### Unmodified and MMG-1-modified C12-200 LNPs efficiently support T_{FH} and GC differentiation and humoral responses

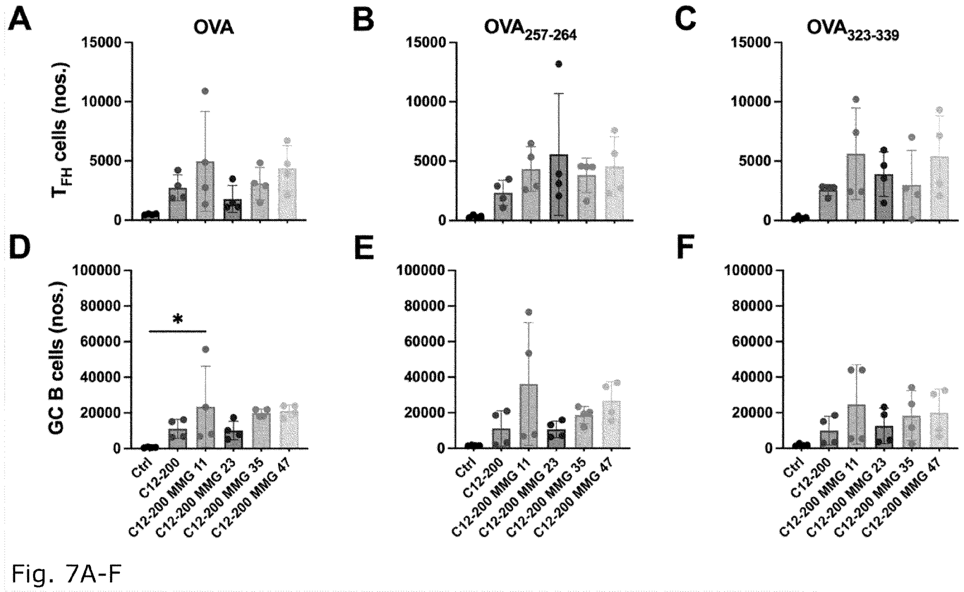

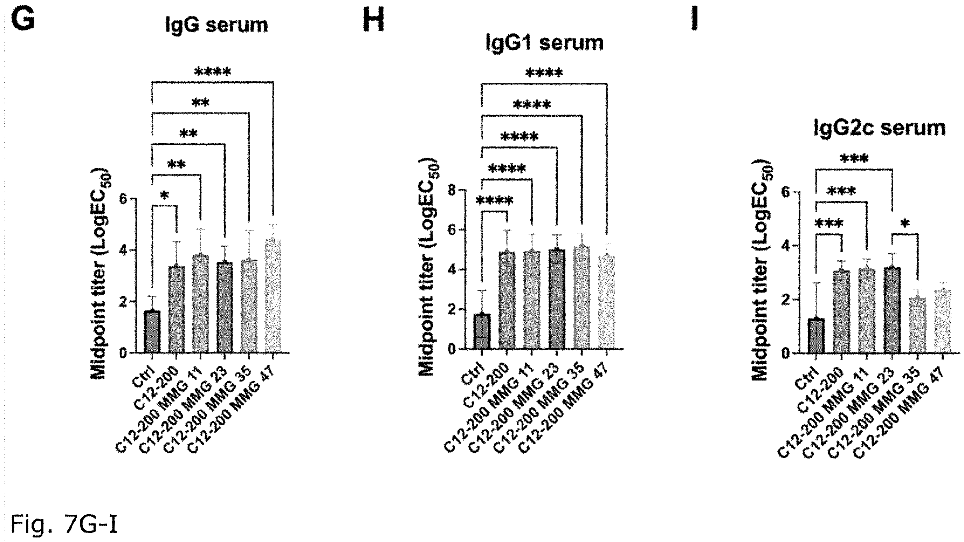

Nucleoside-modified mRNA-LNP vaccines have been reported to induce strong T_{FH} cell generation and GC formation. The inventors therefore measured the numbers of T_{FH} and GC B cells in the LNs draining the s.c. injection site (ILN) of immunised mice **(****Figure 7****).** Animals immunised with *OVA* mRNA-loaded, MMG-1-modified C12-200 LNPs displayed higher numbers of T_{FH} **(**Figure 7A-C) and GC B cells **(**Figure 7D-F) than the un-modified LNPs and naive animals, but the differences were not statistically significant. Of the MMG-1-modified LNPs, C12-200 MMG 11 LNPs had the highest numbers of OVA **(****Figure 7A****),** OVA₂₅₇₋₂₆₄ **(****Figure 7B****),** and OVA₃₂₃₋₃₃₉-specific T_{FH} **(****Figure 7C****),** but the differences were not statistically significant. An almost similar trend was observed for GC B cells **(**Figure 7D-F). To further determine the quality of the antibody responses, the inventors performed ELISA and evaluated midpoint titers of immunoglobulin G (IgG), IgG1, and IgG2c induced in the serum 2 weeks after the booster immunization with mRNA-LNP vaccines. All *OVA* mRNA-loaded LNP vaccines induced significantly higher titers of total IgG **(****Figure 7G****),** IgG1 **(****Figure 7H****),** and IgG2c (Figure 71) than the naive animals. However, there were no differences in the antibody titers between MMG-1 modified versus non-modified C12-200 LNPs. These data indicate that OVA mRNA-loaded C12-200 LNPs can induce functionally variegated humoral responses that are not affected by MMG-1 incorporation into the LNPs.

### Unmodified and MMG-1-modified C12-200 LNPs induce exceptionally high CD8⁺ T cell responses in the spleen

The inventors next evaluated the ability of *OVA* mRNA-loaded LNPs to induce antigen-specific CD4⁺ and CD8⁺ T cells in the spleen. Like LN cells, immune responses in the spleen were assessed by *ex vivo* restimulation of splenocytes with antigen, followed by ICS in combination with tetramer staining and phenotypic marker expression and Boolean gating analysis **(****Figure 8****).** Immunisation with *OVA* mRNA-loaded, MMG-1-modified C12-200 LNPs induced comparable percentages of OVA₂₅₇₋₂₆₄-specific MHC-I (SIINFEKL) (SEQ ID NO.: 6) Tet⁺CD8⁺CD44⁺ T cells that produce IFN-γ than immunisation with *OVA* mRNA-loaded, unmodified C12-200 LNPs, and no other differences were observed for other cytokine-producing T cells **(****Figure 8****).** In general, the average frequencies of these cells were exceptionally high, i.e., 40-50%, and almost two times higher than the frequencies induced in the LNs. Evaluation of the polyfunctional T-cell responses showed a significantly higher percentage (*p < 0.0001) of OVA₂₅₇₋₂₆₄-specific CD8⁺CD44⁺ T cells that co-produced IFN-γ and TNF-a in the spleen of mice vaccinated with *OVA* mRNA-loaded MMG-1-modified C12-200 LNPs, as compared to the percentage for mice vaccinated with *OVA* mRNA-loaded non-modified LNPs (Data not shown).

### Replacing cholesterol with MMG-1 does not influence the CD4⁺ T cell responses in the spleen

We then measured the splenic OVA₃₂₃₋₃₃₉-specific MHC-II (ISQAVHAAHAEINEAGR) (SEQ ID NO.: 7) Tet⁺CD4⁺CD44⁺ T cells. No differences were observed for the cytokine producing OVA₃₂₃₋₃₃₉-specific MHC-II (ISQAVHAAHAEINEAGR) (SEQ ID NO.: 7) Tet⁺CD4⁺CD44⁺ T cells between the LNP groups **(****Figure 9****)** and the average frequencies of these cells was almost the same as that induced in the LNs.

### Conclusion

CD8⁺ T cells are critical for cytotoxic effector functions in infection, cancer and autoimmunity, and they require help from CD4⁺ T cells for effector function and memory responses. Generally, very high MHC-I Tet⁺CD8⁺CD44⁺ T cell responses were displayed in the LNs, as well as the spleen, upon immunisation with all LNP formulations, which were characterised by very well-defined IFN-γ, IL-2, and TNF-a polarised cytokine responses. MMG-1-modified LNPs induced slightly higher frequencies of IFN-γ (12-15%) and TNF-a (7-10%) producing CD8⁺ T cells in the LNs than the non-modified LNPs. However, in the spleen, all LNPs induced approximately 55-60% and 25-35% of IFN-γ⁺ and TNF-α⁺ CD8⁺ T cells, respectively. Both IFN-γ and TNF-a display pro-inflammatory actions, and they are key modulators of cell-mediated immunity against intracellular pathogens and cancer. However, there was no influence of MMG-1 incorporation on the ability of C12-200 LNPs to induce T_{FH} and GC B cell differentiation and induction of humoral responses.

### References

- WO 2021/148511 A1
- Love, K.T., Mahon, K.P., Levins, C.G., Whitehead, K.A., Querbes, W., Dorkin, J.R., Qin, J., Cantley, W., Qin, L.L., Racie, T. et al. (2010) Lipid-like materials for low-dose, in vivo gene silencing. Proc Natl Acad Sci USA, 107, 1864-1869

- Lokras, A., Chakravarty, A., Rades, T., Christensen, D., Franzyk, H., Thakur, A. and Foged, C. (2022) Simultaneous quantification of multiple RNA cargos co-loaded into nanoparticle-based delivery systems. Int J Pharm, 626, 122171.
- Thakur, A., Ingvarsson, P.T., Schmidt, S.T., Rose, F., Andersen, P., Christensen, D. and Foged, C. (2018) Immunological and physical evaluation of the multistage tuberculosis subunit vaccine candidate H56/CAF01 formulated as a spray-dried powder. Vaccine, 36, 3331-3339
- Pastore, G., Carraro, M., Pettini, E., Nolfi, E., Medaglini, D. and Ciabattini, A. (2019) Optimized Protocol for the Detection of Multifunctional Epitope-Specific CD4(+) T Cells Combining MHC-II Tetramer and Intracellular Cytokine Staining Technologies. Front Immunol, 10, 2304.
- Vono, M., Eberhardt, C.S., Auderset, F., Mastelic-Gavillet, B., Lemeille, S., Christensen, D., Andersen, P., Lambert, P.H. and Siegrist, C.A. (2019) Maternal Antibodies Inhibit Neonatal and Infant Responses to Vaccination by Shaping the Early-Life B Cell Repertoire within Germinal Centers. Cell Rep, 28, 1773-1784 e1775.
- Christensen, D., Mortensen, R., Rosenkrands, I., Dietrich, J. and Andersen, P. (2017) Vaccine-induced Th17 cells are established as resident memory cells in the lung and promote local IgA responses. Mucosal Immunol, 10, 260-270.
- Thakur, A., Rodriguez-Rodriguez, C., Saatchi, K., Rose, F., Esposito, T., Nosrati, Z., Andersen, P., Christensen, D., Hafeli, U.O. and Foged, C. (2018) Dual-Isotope SPECT/CT Imaging of the Tuberculosis Subunit Vaccine H56/CAF01: Induction of Strong Systemic and Mucosal IgA and T-Cell Responses in Mice Upon Subcutaneous Prime and Intrapulmonary Boost Immunization. Front Immunol, 9, 2825.

### Sequence listing

| SEQ ID NO. | Sequence name | Sequence* |
|---|---|---|
| 1 | *FLuc* mRNA ORF | See *sequence listing* |
| 2 | *OVA* mRNA ORF | See *sequence listing* |
| 3 | siRNA-TNF-α sense sequence | 5'-pGUCUCAGCCUCUUCUCAUUCCUGct-3' |
| 4 | siRNA-TNF-α antisense sequence | 5'-AGCAGGAAUGAGAAGAGGCUGAGACAU-3' |
| 5 | SARS-CoV2 spike protein | *See sequence listing* |
| 6 | OVA₂₅₇₋₂₆₄ | SIINFEKL |
| 7 | OVA₃₂₃₋₃₃₉ | ISQAVHAAHAEINEAGR |
| 8 | ESAT-6 | *See sequence listing* |
| 9 | Secreted antigen 85b (Ag85B) | *See sequence listing* |
| 10 | Plasmodium falciparum 3D7 circumsporozoite | *See sequence listing* |
| 11 | RSV-F Long mRNA | *See sequence listing* |

| | | |
|---|---|---|
| ^{∗} Lower case letters represent deoxyribonucleotides and *p* represents phosphate residues. | | |

## Claims

1. A lipid nanoparticle (LNP) composition comprising a cationic or cationically ionisable lipid or lipid-like material, a helper lipid, a lipopolymer, and a monomycoloyl glycerol (MMG) analogue,
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-*sn*-glycero-3-phosphocholine (DOPC), 1,2-dioleoyl-*sn*-glycero-3-phospho-(1'-*rac*-glycerol) (DOPG), 1,2-dipalmitoyl-*sn*-glycero-3-phosphocholine (DPPC), 1,2-distearoyl-*sn-*glycero-3-phosphocholine (DSPC), 1,2-dipalmitoyl-*sn*-glycero-3-phosphoglycerol (DPPG), 1,2-dimyristoyl-*sn*-glycero-3-phosphocholine (DMPC), 1,2-diacyl-3-*O*-β-D-galactosyl-*sn*-glycerol (MGDG), 1-stearoyl-2-oleoyl-*sn*-glycero-3-phosphocholine (SOPC), 1,2-diacyl-3-*O*-(α-D-galactosyl1-6)-α-D-galactosyl-*sn-*glycerol (DGDG), and sulfoquinovosyldiacylglycerol (SQDG), or any mixture thereof, and
wherein the lipopolymer is a polyethylene glycol (PEG)- or polysarcosine-lipid conjugate or a PEG- or polysarcosine-lipid like conjugate, or any mixture thereof.

2. The lipid nanoparticle (LNP) composition according to claim 1, wherein the lipopolymer is selected from the group consisting of 1,2-dimyristoyl-*sn*-glycero-3-phosphoethanolamine-N-[methoxy(polyethylene glycol)-2000] (DMPE-PEG₂₀₀₀), 1,2-dimyristoyl-*rac*-glycero-3-methoxypolyethylene glycol-2000 (DMG-PEG₂₀₀₀), 1,2-distearoyl-*sn*-glycero-3-phosphoethanolamine-N-[amino(polyethylene glycol)-2000] (DSPE-PEG₂₀₀₀), 2-[(polyethylene glycol)-2000]-N,N-ditetradecylacetamide (ALC-0159), N-(methylpolyoxyethylene oxycarbonyl)-1,2-dipalmitoyl-*sn*-glycero-3-phosphoethanolamine (DPPE-PEG), 1,2-distearoyl-*rac*-glycerol-3-methoxypolyethylene glycol (DSG-PEG), ceramide-PEG, 1,2-dipalmitoyl-*rac-*glycero-3-methylpolyoxyethylene (DPG-PEG), 1,2-dioleoyl-*rac*-glycerol, methoxypolyethylene glycol (DOG-PEG), 1,2-dioleoyl-*sn*-glycero-3-phosphoethanolamine-N-methylpolyoxyethylene (DOPE-PEG), N-tetradecyl polysarcosine25, N-hexadecyl polysarcosine25, N-octadecyl polysarcosine25, N-dodecyl polysarcosine25, N,N-ditetradecylamine-N-succinyl[methyl(polysarcosine)45], N,N-ditetradecylamine-N-succinyl[methyl(polysarcosine)35], and N,N-ditetradecyl-polysarcosine-25, or any mixture thereof.

3. The lipid nanoparticle (LNP) composition according to any one of claims 1 or 2, wherein the cationic or cationically ionisable lipid or lipid-like material is 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), tetrakis(8-methylnonyl) 3,3',3",3‴-(((methylazanediyl)bis(propane-3,1-diyl))bis(azanetriyl))tetrapropionate (306Oi10), 9-heptadecanyl 8-{(2-hydroxyethyl)[6-oxo-6-(undecyloxy)hexyl]amino}octanoate (SM-102), 1,2-dioleoyl-3-trimethylamonniumpropane (DOTAP), dimethyldioctadecylammonium bromide (DDAB), 1,2-dioleoyl-3-dimethylammonium-chloride (DODAC), 1,2-di-O-octadecenyl-3-trimethylammonium propane (DOTMA), or [(4-hydroxybutyl)azanediyl]di(hexane-6,1-diyl) bis(2-hexyldecanoate) (ALC-0315), preferably C12-200 or SM-102.

4. The lipid nanoparticle (LNP) composition according to any one of the preceding claims, wherein the helper lipid is 1,2-distearoyl-*sn*-glycero-3-phosphocholine (DSPC), 1-stearoyl-2-oleoyl-*sn*-glycero-3-phosphocholine (SOPC) or 1,2-dioleoyl-*sn*-glycero-3-phosphoethanolamine (DOPE), preferably DSPC or DOPE.

5. The lipid nanoparticle (LNP) composition according to any one of the preceding claims, wherein the lipopolymer is 1,2-dimyristoyl-*sn*-glycero-3-phosphoethanolamine-N-[methoxy(polyethylene glycol)-2000] (DMPE-PEG₂₀₀₀), 1,2-distearoyl-rac-glycerol-3-methoxypolyethylene glycol (DSG-PEG), N-tetradecyl polysarcosine25, 1,2-distearoyl-*sn*-glycero-3-phosphoethanolamine-N-[amino(polyethylene glycol)-2000] (DSPE-PEG₂₀₀₀), or 1,2-dimyristoyl-*rac-*glycero-3-methoxypolyethylene glycol-2000 (DMG-PEG₂₀₀₀), preferably DMPE-PEG₂₀₀₀ or DMG-PEG₂₀₀₀.

6. The lipid nanoparticle (LNP) composition according to any one of the preceding claims, wherein the monomycoloyl glycerol (MMG) analogue is selected from the group consisting of MMG-1, MMG-2, MMG-3, MMG-4, MMG-5, MMG-6, and MMG-7, or any mixture thereof, preferably the MMG analogue is MMG-1, MMG-6, and/or MMG-7, more preferably MMG-1.

7. The lipid nanoparticle (LNP) composition according to any one of the preceding claims, wherein said LNP further comprises cholesterol.

8. The lipid nanoparticle (LNP) composition according to any one of the preceding claims, wherein the LNP further comprises at least one nucleic acid.

9. The lipid nanoparticle (LNP) composition according to claim 8, wherein the at least one nucleic acid is selected from the group consisting of messenger RNA (mRNA), self-amplifying RNA, circular RNA (circRNA), plasmid DNA (pDNA), small interfering RNA (siRNA), single guide RNA (sgRNA), guide RNA (gRNA), long non-coding RNA (IncRNA), small activating RNA (saRNA), and splice-switching antisense oligonucleotide (ASO).

10. A vaccine composition comprising the lipid nanoparticle (LNP) composition according to any one of the preceding claims and at least one nucleic acid encoding an antigen.

11. The vaccine composition according to claim 10, wherein the antigen is selected from the group consisting of corona virus antigens, such as SARS-CoV and MERS-CoV antigens, such as SARS-CoV2 spike protein (SEQ ID NO.: 5) or receptor binding domain (RBD), *Mycobacterium tuberculosis* antigens (SEQ ID NO.: 8-9), *Plasmodium falciparum* antigens (SEQ ID NO.: 10), respiratory syncytial virus (RSV) antigens (SEQ ID NO.: 11), Ebolavirus antigens, Marburg virus antigens, Lassa virus antigens, Nipah virus antigens, Zika virus antigens, Crimean-Congo haemorrhagic fever orthonairovirus antigens, human papilloma virus (HPV) antigens, and influenza antigens.

12. The vaccine composition according to any one of claims 10 or 11 for use in the prevention and/or treatment of an infectious disease.

13. A process for obtaining the lipid nanoparticle (LNP) composition according to any one of claims 1-9, said process comprising the steps of:
a) Providing a cationic or cationically ionisable lipid or lipid-like material, a helper lipid, a lipopolymer, a monomycoloyl glycerol (MMG) analogue, and at least one nucleic acid;
b) Dissolving the cationic or cationically ionisable lipid or lipid-like material, the helper lipid, the lipopolymer, and the MMG analogue of step a) in an organic solvent comprising ethanol, preferably absolute ethanol with a purity close to 100%, thereby providing an organic phase;
c) Diluting the at least one nucleic acid of step a) in an aqueous solvent comprising a buffer with a pH within the range of 3 to 7.8, thereby providing an aqueous phase;
d) Mixing the organic phase of step b) with the aqueous phase of step c) to obtain lipid nanoparticles (LNPs) by nanoprecipitation;
e) Performing filtration, preferably tangential flow filtration or dialysis, of the LNPs of step d) to obtain an LNP composition;
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), 9-heptadecanyl 8-{(2-hydroxyethyl)[6-oxo-6-(undecyloxy)hexyl]amino}octanoate (SM-102), [(4-hydroxybutyl)azanediyl]di(hexane-6,1-diyl) bis(2-hexyldecanoate) (ALC-0315), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), dimethyldioctadecylammonium bromide (DDAB), 1,2-dioleoyl-3-dimethylammonium-chloride (DODAC), 1,2-di-O-octadecenyl-3-trimethylammonium propane (DOTMA), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), 1,2-dioleoyl-*sn*-glycero-3-phosphocholine (DOPC), 1,2-dioleoyl-*sn*-glycero-3-phospho-(1'-rac-glycerol) (DOPG), 1,2-dipalmitoyl-*sn*-glycero-3-phosphocholine (DPPC), 1,2-distearoyl-*sn-*glycero-3-phosphocholine (DSPC), 1,2-dipalmitoyl-sn-glycero-3-phosphoglycerol (DPPG), 1,2-dimyristoyl-sn-glycero-3-phosphocholine (DMPC), 1,2-diacyl-3-*O*-β-D-galactosyl-sn-glycerol (MGDG), 1-stearoyl-2-oleoyl-sn-glycero-3-phosphocholine (SOPC), 1,2-diacyl-3-*O*-(α-D-galactosyl1-6)-α-D-galactosyl-*sn-*glycerol (DGDG), and sulfoquinovosyldiacylglycerol (SQDG), or any mixture thereof,
wherein the lipopolymer is a polyethylene glycol (PEG)- or polysarcosine-lipid conjugate or a PEG- or polysarcosine-lipid like conjugate, or any mixture thereof, and
wherein the monomycoloyl glycerol (MMG) analogue is selected from the group consisting of MMG-1, MMG-2, MMG-3, MMG-4, MMG-5, MMG-6, and MMG-7, or any mixture thereof.

14. The process according to claim 13, said process further comprising the step:
f) Concentrating the lipid nanoparticle (LNP) composition using a method selected from the group consisting of filtration, centrifugation, vacuum-assisted centrifugation, or any mixture thereof, preferably filtration.

15. A lipid nanoparticle (LNP) composition obtained using the process of any one of claims 13 or 14.
